# EUROPEAN PATENT APPLICATION

(11) **EP 4 772 226 A2**
(43) Date of publication of application: **08.07.2026**
(21) Application number: 26160780.8
(22) Date of filing: 07.07.2021
(51) Int. Cl.: A61M 16/16

(54) **A RESPIRATORY SUPPORT APPARATUS HAVING A HIGH TEMPERATURE MODE**

(30) Priority: 07.07.2020 US 202063048885 P
(62) Divisional of application: 21838785.0
(71) Applicant: Fisher & Paykel Healthcare Limited, 2013 Auckland (NZ)
(72) Inventor: BURGESS, Russel William, Auckland 2013 (NZ)
(74) Representative: Haseltine Lake Kempner LLP

(57) **Abstract**

According to this disclosure there is provided a respiratory support apparatus configured to provide a gases flow to a patient, the respiratory support apparatus comprising:
a flow generator configured to generate the gases flow;
a humidifier configured to humidify the gases flow; and
a controller.

The apparatus is controlled by the controller to function in at least two modes, being a normal mode and a high-temperature mode. In the high-temperature mode the temperature of gases delivered to the patient is higher than the temperature of gases delivered to the patient when in the normal mode.

In the high-temperature mode, the controller controls one or more parameters of the gases flow to be different to that in the normal mode, whilst providing higher temperatures.

The apparatus is also operative in a cool-down mode in which one or more parameters of the gases flow are controlled.

The apparatus includes safety features to ensure the temperature, dew-point, enthalpy, and/or energy of the gases flow do not exceed safe limits.

## Description

### FIELD OF THE DISCLOSURE

The present disclosure relates to a respiratory support apparatus that has a high temperature mode and is configured to provide a gases flow for inspiration by a patient.

### BACKGROUND

Respiratory support apparatuses are used in various environments, such as hospitals or other medical facilities, to deliver a flow of breathing gases to a patient. The gases flow is typically heated and humidified to aid patient comfort and mitigate drying of the airway. The gases flow is delivered to the patient via an inspiratory conduit and a patient interface. Examples of such respiratory support apparatus include Non-Invasive Ventilation (NIV) apparatus such as CPAP, PEEP, Bi-Level apparatus, and High Flow apparatus.

The patient interface, included in a high flow respiratory support apparatus, typically comprises a nasal cannula. The nasal cannula may comprise one or more prongs that are configured to be received in the nares of the patient. The prongs may be configured to not seal against the nares so that gases (including exhalation gases) can leak around the prongs. The therapy provided by a high flow respiratory support apparatus with a nasal cannula is often referred to as nasal high flow respiratory support or nasal high flow (NHF) therapy.

During NHF therapy, the flow is usually maintained at a set rate within a range (for example, 10 to 100 L/min for adults or 1 to 25 L/min for neonates or children). The gases flow may consist of air, or air supplemented with additional oxygen. During NHF therapy, the temperature of the breathing gas, at the patient interface, can usually be selected from a series of pre-set values (for example, 31 °C, 34 °C, or 37 °C).

Another example of a respiratory support apparatus is a Non-Invasive Ventilation (NIV) apparatus. Non-Invasive Ventilation apparatus may be flow-controlled apparatus (that control to a set flow rate), or pressure-controlled apparatus (that control to a set pressure). Pressure-controlled NIV apparatus may control to a constant pressure (as in CPAP therapy) or to a variable pressure (as in Bi-Level therapy). In Bi-Level NIV therapy, the set pressure during inspiration may be different to the set pressure during expiration.

The patient interface used with a NIV apparatus typically comprises a patient interface that seals, at least partially, against the user's face. Examples of a sealing patient interface include an oral, nasal, or full-face mask comprising a sealing cushion that seals against the patient's face.

### SUMMARY OF DISCLOSURE

The present disclosure stems from some work in trying to provide a respiratory support apparatus that can be used to improve the treatment of patients, and, in some cases, to improve the treatment of patients infected with viruses, such as Human Rhinovirus (HRV) for example.

An apparatus in accordance with this disclosure may improve the treatment of patients having upper respiratory tract viral infections or bacterial infections, for example the human rhinovirus (HRV) or the influenza virus, or other viruses that cause upper respiratory tract infections.

One aspect of the present disclosure is to provide a respiratory support apparatus that is controlled to deliver a high gases flow at relatively high temperature, for example temperatures above 40 °C, and in some cases between 43 and 47 °C.

One aspect of the present disclosure is to provide a respiratory support apparatus that is controlled to deliver a gases flow with a high energy (relative to the energy of gases flows delivered by known respiratory support apparatus). This high energy gases flow may consist of a high temperature gases flow or a high dew-point gases flow (i.e. a gases flow with a high temperature and a high humidity). For example, the temperature of the gases flow may be above 40 °C, and in some cases between 43 and 47 °C. In this example, or another example, the dew-point of the gases flow may be above 40 °C, and in some cases 43 to 47 °C."

Other aspects of this disclosure are to provide a high flow apparatus that is controlled to manage the enthalpy and/or dew-point of the gases flow to help the patient remain safe from excessive enthalpy exposure and thermal injury during and immediately after being exposed to a high flow of high-temperature gases. The enthalpy and/or dew-point can be measured at the patient interface or calculated from measurements made elsewhere in the gases flow path between the flow generator and the patient interface.

Aspects of this disclosure are to providing an apparatus that is operative according to a normal mode, and according to a high-temperature mode in which respiratory gases are delivered to the patient at relatively high temperatures, the apparatus being further operative according to one or more safety algorithms. The safety algorithm(s) may be configured to control one or more properties of the respiratory gases to be within safe levels during the high-temperature mode. The safety algorithm(s) may be configured to control one or more properties of the respiratory gases to be within safe levels after the high-temperature mode has terminated, but before the normal mode resumes.

The present disclosure provides an apparatus that is controlled to deliver a gases flow at a relative high temperature so as to increase the temperature of the gases supplied to the patient, and/or to increase the dew-point of the gases supplied to the patient. The dew-point of the gases supplied to the patient will vary in dependence upon the humidity of the gases. The apparatus may be controlled to operate in a high temperature mode, in which either the temperature of the gases supplied to the patient is relatively high, or both the temperature and the dew-point of the gases supplied to the patient are relatively high.

In an apparatus in accordance with this disclosure, the gases temperature and/or dew-point refers to the temperature and/or dew-point of the gases as supplied to the patient (i.e. at or in the patient interface). The temperature at the patient could be measured using one or more sensors at the patient. For example, these one or more sensors could be at the patient end of an inspiratory conduit, or at or in the patient interface itself. Alternatively, the temperature could be measured elsewhere in the apparatus. For example, the temperature could be measured at the humidifier, at the humidifier outlet, or at some other point between the humidifier and the patient. If the temperature is measured distal from the patient (at a humidifier outlet for example), a controller of the apparatus may be configured to calculate how the temperature might change between the point of measurement and the patient. The controller may be configured to control a heater of a heated inspiratory conduit to try to maintain the temperature of the gases before the gases reach the patient. In other words, the controller may control the conduit heater to compensate for any calculated temperature drop along the inspiratory conduit.

The controller may control the conduit heater based on feedback from one or more patient end sensors, such as a temperature sensor and/or humidity sensor, at a patient end of the conduit. The patient end sensor(s) may be located at the patient end of the conduit, for example in a cuff or connector at the end of the conduit. The patient end sensor(s) may be located in the patient interface. The controller may be configured to determine a temperature drop from the end of the inspiratory conduit to the patient, for example a 3°C drop.

Apparatus in accordance with this disclosure may be controlled to deliver a gases flow with a high temperature and a high dew-point (i.e. a gases flow with a high temperature and a relative humidity sufficiently high for the gases flow to have a high dew-point).

In one example the high dew-point comprises a temperature above 40°C and humidity of over 80%.

Apparatus in accordance with this disclosure can be operative according to a high-temperature mode wherein the gases flow has a higher peak temperature and/or a higher dew-point than when in a normal mode. The normal mode is a therapy mode in which respiratory gases are delivered to the patient, but not at high temperature or high dew-point.

An aspect of the present disclosure is a safety algorithm configured to control a respiratory apparatus during and after a high-temperature mode in which gases are delivered to the patient at high temperature and high dew-point. In some aspects, this safety algorithm:
a) limits the flow rates that can be used during a high-temperature mode; and/or
b) limits the duration of a high-temperature mode; and/or
c) controls the process by which the apparatus returns from a high-temperature mode to a normal mode, in which gas is delivered to the patient at standard flow rates and lower temperatures.

According to one aspect of this disclosure there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient when in the normal mode;
wherein in the normal mode the controller controls the flow generator to generate the gases flow at a flow rate within a first flow rate range that is limited by a first peak flow rate; and
wherein in the high-temperature mode, the controller controls the flow generator to generate the gases flow at a flow rate within a second flow rate range that is limited by a second peak flow rate, the second peak flow rate being lower than the first peak flow rate of the normal mode.

According to one aspect of this disclosure there is provided a non-invasive ventilation respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient when in the normal mode;
wherein in the normal mode the controller controls the flow generator to generate the gases flow at a flow rate within a first flow rate range that is limited by a first peak flow rate; and
wherein in the high-temperature mode, the controller controls the flow generator to generate the gases flow at a flow rate within a second flow rate range that is limited by a second peak flow rate, the second peak flow rate being lower than the first peak flow rate of the normal mode.

According to one aspect of this disclosure there is provided a high flow respiratory support apparatus configured to provide a high gases flow to a patient, the respiratory support apparatus comprising:
a flow generator configured to generate the gases flow;
a humidifier configured to humidify the gases flow;
a controller, the apparatus being controlled by the controller to function in at least two high flow modes, being a normal mode and a high-temperature mode, wherein when in the high-temperature mode the temperature of gases delivered to the patient is higher than the temperature of gases delivered to the patient when in the normal mode;
wherein in the normal mode the controller controls the flow generator to generate the gases flow at a flow rate within a first flow rate range that is limited by a first peak flow rate; and wherein in the high-temperature mode, the controller controls the flow generator to generate the gases flow at a flow rate within a second flow rate range that is limited by a second peak flow rate, the second peak flow rate being lower than the first peak flow rate of the normal mode.

The first flow rate range may be limited by a first minimum flow rate, and the second flow rate range is limited by a second minimum flow rate.

The magnitude of the change between the first flow rate range and the second flow rate range may be controlled so as to be below a predetermined threshold.

The minimum flow rate of the second flow rate range may be equal to or less than the minimum flow rate of the first flow rate range when in normal mode.

The second flow rate range may be narrower than the first flow rate range.

The second flow rate range may be narrower than the first flow rate range by between 1 and 70 L/min, preferably between 5 and 60 L/min and more preferably between 10 and 60 L/min.

The first flow rate range may be between 0 L/min to 100L/min. The second flow rate range may be between 20 - 80 L/min, more preferably 30 - 60L/min.

The second flow rate may be less than the first flow rate by between 5 and 30L/min, or 10 and 25L/min, or by substantially 15L/min, or by substantially 10L/min.

When in the high-temperature mode the gases flow to the patient may be in a temperature range of 41 to 50 °C, preferably 43 to 47 °C.

When in the high-temperature mode the controller may limit the duration of the high-temperature mode to a maximum duration.

The apparatus may be controlled by the controller in a further mode, being a cool-down mode, the cool-down mode being operative after the high-temperature mode, such that when in the cool-down mode, any one or more of:
a) the flow rate is controlled to a flow rate set point, wherein the rate of change of the flow rate from the flow rate used during high-temperature mode to the flow rate set point is controlled to be below a predetermined threshold;
b) the electrical power supplied to the humidifier heater is reduced from that used during normal mode and/or high-temperature mode; and/or
c) a temperature set point of the gases flow delivered to the patient is reduced from that during high-temperature mode.

The electrical power may be reduced, for example, by controlling PWM, duty cycle, voltage, and/or current.

The electrical power may be reduced by a predetermined amount, and/or may be reduced to zero.

According to another aspect of this disclosure, there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode;
wherein in the high-temperature mode the controller limits the duration of the high-temperature mode to a maximum duration. This duration may be a set duration in which the apparatus is controlled to operate in high-temperature mode for a predetermined period of time. The user may exit high-temperature mode at any time up to the maximum duration.

The flow generator may comprise one or more blowers, each comprising a motor and an impeller. The blower(s) may be mounted in a housing of the apparatus. The humidifier may also be mounted in or on the housing of the apparatus.

According to another aspect of this disclosure, there is provided a respiratory support apparatus configured to provide a gases flow to a patient, the respiratory support apparatus comprising:
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode;
wherein in the high-temperature mode the controller limits the duration of the high-temperature mode to a maximum duration. This duration may be a set duration in which the apparatus is controlled to operate in high-temperature mode for a predetermined period of time. The user may exit high-temperature mode at any time up to the maximum duration.

The apparatus may comprise a gas inlet configured to receive one or more gases from an external gas source. The apparatus may comprise a plurality of gas inlets. The external gas source could comprise a source of oxygen for example. The external gas source could comprise a wall source, such as a oxygen wall supply in a hospital. The external gas source could comprise a tank or canister of compressed gas(es).

The controller may limit the duration of the high-temperature mode to a set duration using a signal generated by a timer.

The set duration may be between 15 and 180 minutes, preferably 20 and 150 minutes, and more preferably between 30 and 120 minutes.

The controller may limit the duration of the high-temperature mode to a set duration using a signal generated by a counter.

The apparatus may generate the signal.

The apparatus may comprise a further controller, configured to generate the signal.

The apparatus may receive the signal from a remote device.

The apparatus may be controlled by the controller in a further mode, being a cool-down mode, the cool-down mode being operative after the high-temperature mode, and on expiry of the set duration.

When in the cool-down mode, any one or more of:
the flow rate may be controlled to a flow rate set point, wherein the rate of change of the flow rate from the flow rate used during high-temperature mode to the flow rate set point is controlled to be below a predetermined threshold;
the electrical power supplied to the humidifier heater may be reduced from that used during normal mode and/or high-temperature mode; and/or
a temperature set point of the gases flow delivered to the patient may be reduced from that during high-temperature mode.

The electrical power supplied to the humidifier heater may be varied by varying any one or more of PWM, duty cycle, voltage and/or current.

In cool-down mode, the apparatus may be controlled to a gases flow temperature set point of 40 °C or less.

In cool-down mode, the apparatus may be controlled to generate a gases flow at a flow rate within a range of flow rates that are generated in the normal mode.

In cool-down mode, the apparatus may be controlled such that the enthalpy and/or dew-point of the gases flow is reduced from the enthalpy and/or dew-point of the gases flow in high temperature mode.

When in the high-temperature mode the gases flow to the patient may be in a temperature range of 41 to 50 °C, or 43 to 47 °C, or 43 to 45 °C.

According to a further aspect of this disclosure there is provided a respiratory support apparatus configured to provide a gases flow to a patient, the respiratory support apparatus comprising:
a flow generator configured to generate the gases flow;
a humidifier configured to humidify the gases flow, the humidifier comprising a humidifier heater;
a controller, the apparatus being controlled by the controller to function in at least two flow modes, being a normal mode and a high-temperature mode, wherein when in the high-temperature mode the temperature of gases delivered to the patient is higher than the temperature of gases delivered to the patient in the normal mode;
the apparatus further being controlled by the controller in a further mode, being a cool-down mode, the cool-down mode being operative after the high-temperature mode, such that when in the cool-down mode, any one or more of:
   a) the flow rate is controlled to a flow rate set point, wherein the rate of change of the flow rate from the flow rate used during high-temperature mode to the flow rate set point is controlled to be below a predetermined threshold;
   b) the electrical power supplied to the humidifier heater is reduced from that used during normal and/or high-temperature mode; and/or
   c) a temperature set point of the gases flow delivered to the patient is reduced from that during high-temperature mode.

According to a further aspect of this disclosure there is provided a high flow respiratory support apparatus configured to provide a high gases flow to a patient, the respiratory support apparatus comprising:
a flow generator configured to generate the gases flow;
a humidifier configured to humidify the gases flow, the humidifier comprising a humidifier heater;
a controller, the apparatus being controlled by the controller to function in at least two high flow modes, being a normal mode and a high-temperature mode, wherein when in the high-temperature mode the temperature of gases delivered to the patient is higher than the temperature of gases delivered to the patient in the normal mode;
the apparatus further being controlled by the controller in a further mode, being a cool-down mode, the cool-down mode being operative after the high-temperature mode, such that when in the cool-down mode, any one or more of:
a) the flow rate is controlled to a flow rate set point, wherein the rate of change of the flow rate from the flow rate used during high-temperature mode to the flow rate set point is controlled to be below a predetermined threshold;
b) the electrical power supplied to the humidifier heater is reduced from that used during normal and/or high-temperature mode; and/or
c) a temperature set point of the gases flow delivered to the patient is reduced from that during high-temperature mode.

The cool-down mode may be automatically selected by the controller, in response to one or more predetermined conditions of operation of the apparatus.

The one or more predetermined conditions of operation may be selected from any one or more of:
a) a predetermined period of time, as determined by a timer;
b) a predetermined number of counts, as determined by a counter;
c) a gases flow dew-point being exceeded;
d) a gases flow enthalpy being exceeded;
e) the user exiting high-temperature mode.

The controller may return the apparatus to normal mode, after the cool-down mode.

The controller may return the apparatus to normal mode, after a predetermined period of time of the cool-down mode, which may be in the range of 15 to 60 minutes, preferably 30 minutes.

The controller may return the apparatus to normal mode in dependence upon the temperature of the gases flow.

The controller may return the apparatus to normal mode in dependence upon the temperature of the gases flow being less than a predetermined temperature.

The controller may return the apparatus to normal mode in dependence upon the dew-point of the gases flow.

The controller may return the apparatus to normal mode in dependence upon the enthalpy of the gases flow.

When in the high-temperature mode the gases flow to the patient may be in a temperature range of 41 to 50 °C, 43 to 47 °C, preferably 43 to 45 °C.

According to another aspect of this disclosure there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode;
wherein the controller controls the apparatus to restrict a characteristic of the gases flow during high-temperature mode, and/or immediately after termination of high-temperature mode, such that the enthalpy and/or the dew-point of the gases flow is below a predetermined level. As noted above, the temperature, enthalpy, and/or dew-point of the gases flow may be measured at or near the patient interface or measured elsewhere in the inspiratory gases flow path between the flow generator and the patient interface.

According to another aspect of this disclosure there is provided a respiratory support apparatus configured to provide a gases flow to a patient, the respiratory support apparatus comprising:
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode;
wherein the controller controls the apparatus to restrict a characteristic of the gases flow during high-temperature mode, and/or immediately after termination of high-temperature mode, such that the enthalpy and/or the dew-point of the gases flow is below a predetermined level. As noted above, the temperature, enthalpy, and/or dew-point of the gases flow may be measured at or near the patient interface or measured elsewhere in the inspiratory gases flow path between the flow generator and the patient interface.

When in the high-temperature mode the gases flow to the patient may be in a temperature range of 41 to 50 °C, 43 to 47 °C, preferably 43 to 45 °C.

According to a further aspect of this disclosure there is provided a respiratory support apparatus configured to provide a gases flow to a patient, the respiratory support apparatus comprising:
a flow generator configured to generate the gases flow;
a humidifier configured to humidify the gases flow, the humidifier comprising a humidifier heater;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode; wherein when in the high-temperature mode the gases flow to the patient is in a temperature range of 41 to 50 °C or 43 to 47 °C.

According to a further aspect of this disclosure there is provided a respiratory support apparatus configured to provide a gases flow to a patient, the respiratory support apparatus comprising:
a humidifier configured to humidify the gases flow, the humidifier comprising a humidifier heater;
a controller, the apparatus being controlled by the controller to function in at least two modes, being a normal mode and a high-temperature mode, wherein when in the high-temperature mode the temperature of gases delivered to the patient is higher than the temperature of gases delivered to the patient in normal mode; wherein when in the high-temperature mode the gases flow to the patient is in a temperature range of 41 to 50 °C or 43 to 47 °C.

According to a further aspect of this disclosure there is provided a respiratory support apparatus configured to provide a gases flow to a patient, the respiratory support apparatus comprising:
a flow generator configured to generate the gases flow;
a humidifier configured to humidify the gases flow, the humidifier comprising a humidifier heater;
a controller, the apparatus being controlled by the controller to function in at least two modes, being a normal mode and a high-temperature mode, wherein when in the high-temperature mode the dew-point of gases delivered to the patient is higher than the dew-point of gases delivered to the patient in normal mode; wherein when in the high-temperature mode the gases flow to the patient is in a dew-point range of 43 to 47 °C.

According to a further aspect of this disclosure there is provided a respiratory support apparatus configured to provide a gases flow to a patient, the respiratory support apparatus comprising:
a humidifier configured to humidify the gases flow, the humidifier comprising a humidifier heater;
a controller, the apparatus being controlled by the controller to function in at least two modes, being a normal mode and a high-temperature mode, wherein when in the high-temperature mode the dew-point of gases delivered to the patient is higher than the dew-point of gases delivered to the patient in normal mode; wherein when in the high-temperature mode the gases flow to the patient is in a dew-point range of 43 to 47 °C.

The apparatus may comprise any one or more of:
a) a patient interface configured to be mounted on the patient and to receive the humidified gases flow from the humidifier;
b) a patient circuit comprising at least one patient conduit configured to be connected to an outlet of the humidifier, and to receive the humidified gases flow from the humidifier;
c) headgear configured to mount a patient interface on the patient's head.

The patient interface may comprise an unsealed interface. The patient interface may comprise a nasal cannula or a tracheal interface such as a tracheal tube.

The patient interface may comprise a sealing interface.

The apparatus may comprise a user interface arranged to a receive an input from the user to control the apparatus. The user interface may be a graphic user interface, and preferably a touch-screen graphic user interface. The normal and high-temperature modes may be user selectable, via the user interface.

The user interface may be configured to present a countdown timer. The user interface may also display a button or interactive element that can re-start the timer and the high temperature mode.

The apparatus may be configured to limit the number of times a user can consecutively activate the high temperature mode.

The apparatus may be controlled to provide a cool-down mode, after termination of the high-temperature mode.

The cool-down mode may be automatically selected by the controller, in response to one or more predetermined conditions of operation of the apparatus.

The one or more predetermined conditions of operation may be selected from any one or more of:
a) a predetermined period of time, as determined by a timer;
b) a predetermined number of counts, as determined by a counter;
c) a gases flow dew-point being exceeded;
d) a gases flow enthalpy being exceeded;
e) the user exiting the high-temperature mode.

The apparatus may be controlled, after the cool-down mode, to return to the normal mode.

When in normal mode, the gases flow may be controlled to have any one or more of the following characteristics:
a) a dew-point in the range of 31 °C - 37 °C;
b) a temperature in the range of 31 °C - 37 °C;
c) a flow rate in the range of 10 L/min - 70 L/min.

When in high-temperature mode, the gases flow may be controlled to have any one or more of the following characteristics:
a) a dew-point in the range of 40 °C - 47 °C;
b) a temperature in the range of 41 °C - 50 °C;
c) a flow rate in the range of 30 L/min - 40 L/min;
d) a minimum flow rate that is a predetermined amount less than the minimum flow rate in the normal mode;
e) a minimum flow rate that is a predetermined amount more than the minimum flow rate in the normal mode; and
f) a maximum flow rate that is a predetermined amount less than the maximum flow rate in the normal mode.

The flow rate range during high-temperature mode may be related to the flow rate range used during normal mode. For example, the flow rate range during high-temperature mode may have the same flow-range mid-point.

According to one aspect of this disclosure there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the temperature of gases delivered to the patient is higher than the temperature of gases delivered to the patient when in the normal mode;
wherein in the normal mode the controller controls the flow generator to generate the gases flow at a flow rate within a first flow rate range that is limited by a first peak flow rate; and wherein in the high-temperature mode, the controller controls the flow generator to generate the gases flow at a flow rate within a second flow rate range that is limited by a second peak flow rate, the second peak flow rate being lower than the first peak flow rate of the normal mode; wherein in the high-temperature mode the controller limits the duration of the high-temperature mode to a maximum duration;
the apparatus further being controlled by the controller in a further mode, being a cool-down mode, the cool-down mode being operative after the high-temperature mode, such that when in the cool-down mode, any one or more of:
   a) the flow rate is controlled to a flow rate set point, wherein the rate of change of the flow rate from the flow rate used during high-temperature mode to the flow rate set point is controlled to be below a predetermined threshold;
   b) the electrical power supplied to the humidifier heater is reduced from that used during normal and/or high-temperature mode; and/or
   c) a temperature set point of the gases flow delivered to the patient is reduced from that during high-temperature mode.

An apparatus in accordance with this disclosure may be a high flow apparatus configured to deliver a high flow of gases to the patient.

The high flow apparatus may be configured to be used with, or comprise, a non-sealing patient interface. An example of a non-sealing patient interface includes a nasal cannula having one or more non-sealing nasal prongs. The non-sealing nasal prongs do not seal against the patient's nares.

An apparatus in accordance with this disclosure may be a non-invasive ventilation apparatus.

The non-invasive ventilation apparatus may be configured to be used with, or comprise, a sealing patient interface. Examples of a sealing patient interface include an oral, nasal, or full-face mask comprising a sealing cushion that seals against the patient's face.

An apparatus in accordance with this disclosure may be a pressure support apparatus such as a CPAP, or Bi-Level apparatus for example.

According to an aspect of this disclosure there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in first and second modes, wherein when in the first mode, the gases are delivered to the patient at a higher dew-point and/or a higher average temperature than when in the second mode.

According to an aspect of this disclosure there is provided a respiratory support apparatus configured to provide a gases flow to a patient, the respiratory support apparatus comprising:
a humidifier configured to humidify the gases flow;
a controller, the apparatus being controlled by the controller to function in first and second modes, wherein when in the first mode, the gases are delivered to the patient at a higher dew-point and/or a higher average temperature than when in the second mode.

According to an aspect of this disclosure there is provided a respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode the apparatus is operable in a first flow range and when in the normal mode the apparatus is operable in a second flow range, wherein the second flow range is larger than the first flow range, and wherein the apparatus is controlled in the high flow mode for a predetermined duration.

The time duration may for example be less than 120 minutes, less than 90 minutes, less than 60 minutes, less than 45 minutes, or less than 30 minutes.

The second flow range may be larger in terms of the peak flow rate of the second range, and/or the breadth of flow rates of the second range.

According to another aspect of this disclosure there is provided a method of providing respiratory support to a patient, using an apparatus according to any one or more of the above statements.

The respiratory support apparatus may comprise an inspiratory conduit configured to provide an inspiratory gases flow path for the gases flow between the flow generator outlet and the patient interface.

The flow generator may comprise a blower. The flow generator may comprise a plurality of blowers. One of the blowers of the plurality of blowers may be configured to generate the gases flow, and another of the blowers of the plurality of blowers may be configured to force expiratory gases along the expiratory conduit, for example via generation of suction force in the expiratory conduit.

The apparatus may comprise a housing. The flow generator may be located in the housing. The humidifier may be located in the housing. Both the flow generator and the humidifier may be located in the same housing.

According to another aspect of this disclosure there is provided a respiratory system comprising:
the respiratory support apparatus of any one of the above statements;
a) a patient interface; and
b) an inspiratory conduit. The inspiratory conduit may comprise a heater such as a heater wire, configured to heat gases in the conduit.

Note that the terms "patient" and "user" are both used in this document. The term "patient" refers to the person that is receiving the gases flow from the high flow apparatus. The term "user" refers to the person who is interacting with the apparatus' controller, which may include a graphical user interface (GUI) for example. The user and the patient can be the same person, or different people.

Features from one or more embodiments or configurations may be combined with features of one or more other embodiments or configurations. Additionally, more than one embodiment may be used together during a process of respiratory support of a patient.

The term 'comprising' as used in this specification means 'consisting at least in part of'. When interpreting each statement in this specification that includes the term 'comprising', features other than that or those prefaced by the term may also be present. Related terms such as 'comprise' and 'comprises' are to be interpreted in the same manner.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

It should be understood that alternative embodiments or configurations may comprise any or all combinations of two or more of the parts, elements or features illustrated, described or referred to in this specification.

This invention may also be said broadly to consist in the parts, elements and features referred to or indicated in the specification of the application, individually or collectively, and any or all combinations of any two or more said parts, elements, or features.

To those skilled in the art to which the invention relates, many changes in construction and widely differing embodiments and applications of the invention will suggest themselves without departing from the scope of the invention as defined in the appended claims. The disclosures and the descriptions herein are purely illustrative and are not intended to be in any sense limiting. Where specific integers are mentioned herein which have known equivalents in the art to which this invention relates, such known equivalents are deemed to be incorporated herein as if individually set forth.

### BRIEF DESCRIPTION OF THE DRAWINGS

**Figure 1A** shows in diagrammatic form a respiratory support apparatus.
**Figure 1B** illustrates a sensing circuit board including a flow rate sensor that may be used in a respiratory support apparatus.
**Figure 2** is a flow diagram of control steps provided by a controller of a respiratory support apparatus including a high-temperature mode, in accordance with this disclosure.
**Figure 3** is a flow diagram of control steps provided by a controller of a respiratory support apparatus including a cool-down mode, in accordance with this disclosure.
**Figures 4a to 4c** show possible screens of a graphic user interface that may comprise part of a respiratory support apparatus in accordance with this disclosure.
**Figure 5** is a further possible screen of a graphic user interface that may comprise part of a respiratory support apparatus in accordance with this disclosure.
**Figure 6** is a graph showing how the enthalpy of the gas provided to the patient by a respiratory support apparatus can change with respect to time.
**Figure 7** shows in diagrammatic form a respiratory support apparatus in accordance with this disclosure, in communication with a remote management system.
**Figure 8** is an example electrical circuit incorporating a patient sensor of an apparatus in accordance with this disclosure.

### DETAILED DESCRIPTION

This disclosure relates to a respiratory support apparatus that is configured to safely deliver a gases flow at a high temperature to a patient. Introducing a flow of humidified gases into a patient's nares at a high temperature may lessen the symptoms associated with viruses such as the Human Rhinovirus (which can cause the common cold), Influenza, and/or any other viruses that can reside in the upper airways of the patient or enter the human body through the upper airways of the patient. This lessening of symptoms may be due to the increased thermal energy that is transferred to the patient's airways by the flow of high-temperature gases. In accordance with aspects of this disclosure, the flow of gases may be a high flow of gases, for example at flow rates suitable for NHF.

A respiratory support apparatus in accordance with this disclosure can help overcome a problem which is that the patient may experience thermal injury, during or immediately after the therapy. This thermal injury may occur if the enthalpy (or dew-point) of the gases flow is too high and/or if the high-temperature therapy session lasts for too long.

Further implementing detail of an apparatus in accordance with this disclosure is provided below.

However, in summary, an aspect of this disclosure provides a high flow respiratory support apparatus which is operative between a normal high flow mode, and a high-temperature high flow mode. The high temperature mode can be considered to be a high-energy mode in which the energy of the gases flow to the patient is increased, as compared to the gases flow delivered during normal therapy. The apparatus comprises a flow generator. The apparatus is controlled according to the following high-level control stages:
a) Initiation of High-Temperature Mode
   - The peak gases flow is reduced, as compared to the peak gases flow in normal mode. Alternatively, or additionally, the flow range of the gases flow is reduced, as compared to the flow range of the gases flow in normal mode. The apparatus may alter the gases flow automatically.
b) Operation of High-Temperature Mode
   - The timer or counter generates a control signal to limit the maximum duration of high-temperature mode.
c) Post Operation of High-Temperature Mode - First Stage
   - The gases flow rate is slowly increased (or decreased) towards its post-high-temperature-mode set point. The apparatus may increase (or decrease) the gases flow automatically.
   - The electrical power delivered to a gases heater, for example a humidifier heater, is reduced. For example, the pulse width modulation (PWM) of the humidifier heater plate may be set to zero for a set time.
   - The gases flow temperature downstream of a humidifier of the apparatus is limited, for a set time. The controller may limit the maximum temperature set point, for a predetermined time.
d) Post Operation of High-Temperature Mode - Subsequent Stage
   - The electrical power that may be supplied to the gases heater is increased. The power that is supplied to the heater could be varied in a number of ways. For example, the PWM of the humidifier heater (which may be a heater plate) could be increased back up to the PWM used when the apparatus is in normal mode. The power supplied to the gases heater could also (or alternatively) be varied by varying the duty cycle of the gases heater, or by varying the voltage and/or current supplied to the gases heater.
   - The gases flow temperature (as measured at a specific point in the flow path) is slowly increased towards its post-high-temperature-mode set point (for that specific point in the flow path). The gases flow temperature can be measured at any point downstream of a humidifier (e.g. at the patient interface). In some examples it is convenient to measure the gases flow temperature at the end of the hose that delivers the gases flow from the humidifier to the patient interface (this measurement is known as the end-of-hose or EoH temperature).

An aspect of this disclosure provides a respiratory support apparatus, such as an NIV apparatus, which is operative between a normal mode, and a high-temperature mode. The apparatus comprises a flow generator. The apparatus is controlled according to the following high-level control stages:
a) Initiation of High-Temperature Mode
   - The peak gases flow is reduced, as compared to the peak gases flow in normal mode. Alternatively, or additionally, the flow range of the gases flow is reduced, as compared to the flow range of the gases flow in normal mode. The apparatus may alter the gases flow automatically.
   - Alternatively, the maximum and/or minimum pressure is altered.
   - Alternatively, the apparatus 10 may control the flow generator to deliver a constant pressure during high-temperature mode.
b) Operation of High-Temperature Mode
   - The timer or counter generates a control signal to limit the maximum duration of high-temperature mode.
c) Post Operation of High-Temperature Mode - First Stage
   - The gases flow rate is slowly increased (or decreased) towards its post-high-temperature-mode set point. The apparatus may increase the gases flow automatically.
   - The electrical power delivered to a gases heater, for example a humidifier heater, is reduced. For example, the PWM of the humidifier heater plate may be set to zero for a set time.
   - The gases flow temperature downstream of a humidifier of the apparatus is limited, for a set time. The controller may limit the maximum temperature set point, for a predetermined time.
d) Post Operation of High-Temperature Mode - Subsequent Stage
   - The electrical power that may be supplied to the gases heater is increased. The power that is supplied to the heater could be varied in a number of ways. For example, the PWM of the humidifier heater (which may be a heater plate) could be increased back up to the normal PWM used when the apparatus is in normal mode. The power supplied to the gases heater could also (or alternatively) be varied by varying the duty cycle of the gases heater, or by varying the voltage and/or current supplied to the gases heater.
   - The gases flow temperature (as measured at a specific point in the flow path) is slowly increased towards its post-high-temperature-mode set point (for that specific point in the flow path). The gases flow temperature can be measured at any point downstream of a humidifier (e.g. at the patient interface). In some examples it is convenient to measure the gases flow temperature at the end of the hose that delivers the gases flow from the humidifier to the patient interface (this measurement is known as the end-of-hose or EoH temperature).

An aspect of this disclosure provides a respiratory support apparatus, which is operative between a normal mode, and a high-temperature mode, and where the apparatus is controlled according to the following high-level control stages:
Initiation of High-Temperature Mode
   - The peak gases flow is reduced, as compared to the peak gases flow in normal mode. Alternatively, or additionally, the flow range of the gases flow is reduced, as compared to the flow range of the gases flow in normal mode. If the apparatus does not comprise a flow generator, or the flow generator is not used, the apparatus may receive a gases flow from one or more gas sources to which the apparatus is connected. In such an embodiment, the apparatus may receive a reduced gases flow that the user has manually adjusted, for example by closing or opening a flow control valve of the gases source. In such an embodiment, the apparatus may be configured to generate one or more reminders, to indicate to the user to reduce the gas(es) flow from the gas(es) source(s) when High-Temperature Mode is initiated. Operation of High-Temperature Mode
   - The timer or counter generates a control signal to limit the maximum duration of high-temperature mode.
Post Operation of High-Temperature Mode - First Stage
   - The gases flow rate is slowly increased (or decreased) towards its post-high-temperature-mode set point. The apparatus may increase the gases flow automatically.
   - The electrical power delivered to a gases heater, for example a humidifier heater, is reduced. For example, the PWM of the humidifier heater plate may be set to zero for a set time.
   - The gases flow temperature downstream of a humidifier of the apparatus is limited, for a set time. The controller may limit the maximum temperature set point, for a predetermined time.
Post Operation of High-Temperature Mode - Subsequent Stage
   - The electrical power that may be supplied to the gases heater is increased. The power that is supplied to the heater could be varied in a number of ways. For example, the PWM of the humidifier heater (which may be a heater plate) could be increased back up to the normal PWM used when the apparatus is in normal mode. The power supplied to the gases heater could also (or alternatively) be varied by varying the duty cycle of the gases heater, or by varying the voltage and/or current supplied to the gases heater.
   - The gases flow temperature (as measured at a specific point in the flow path) is slowly increased towards its post-high-temperature-mode set point (for that specific point in the flow path). The gases flow temperature can be measured at any point downstream of a humidifier (e.g. at the patient interface). In some examples it is convenient to measure the gases flow temperature at the end of the hose that delivers the gases flow from the humidifier to the patient interface (this measurement is known as the end-of-hose or EoH temperature).

### Respiratory Support Apparatus

It is known to provide a respiratory support apparatus. One example of such a respiratory support apparatus is a high flow respiratory support apparatus, which comprises a patient interface in the form of a nasal cannula. The nasal cannula comprises prongs that are received in the nares of the patient. In such an apparatus the patient typically exhales directly to atmosphere via their mouth, and/or via leak around the prongs of the cannula. In such an example, such a respiratory support apparatus can be controlled to a user-defined flow rate, so that it generates an inspiratory gases flow at a flow rate of, for example, around 50-60 L/min. In an alternative form, a high flow respiratory support apparatus may comprise a tracheal patient interface to deliver high flow through, for example, a tracheal tube inserted into the neck of a patient.

Another example of a respiratory support apparatus is a NIV apparatus, which comprises a patent interface in the form of a sealing interface such as a full-face mask, that seals around the nose and mouth of the patient. In such an example such a respiratory support apparatus can be controlled to a user-defined pressure. The pressure can generate an inspiratory gases flow at a flow rate of, for example, around 1-40 L/min. An NIV apparatus may be provided in accordance with this disclosure, operative according to normal, high-temperature, and cool-down modes, where a high temperature inspiratory gases flow is provided to the patient. The inspiratory gases flow during each mode may be controlled by controlling the pressure of that flow.

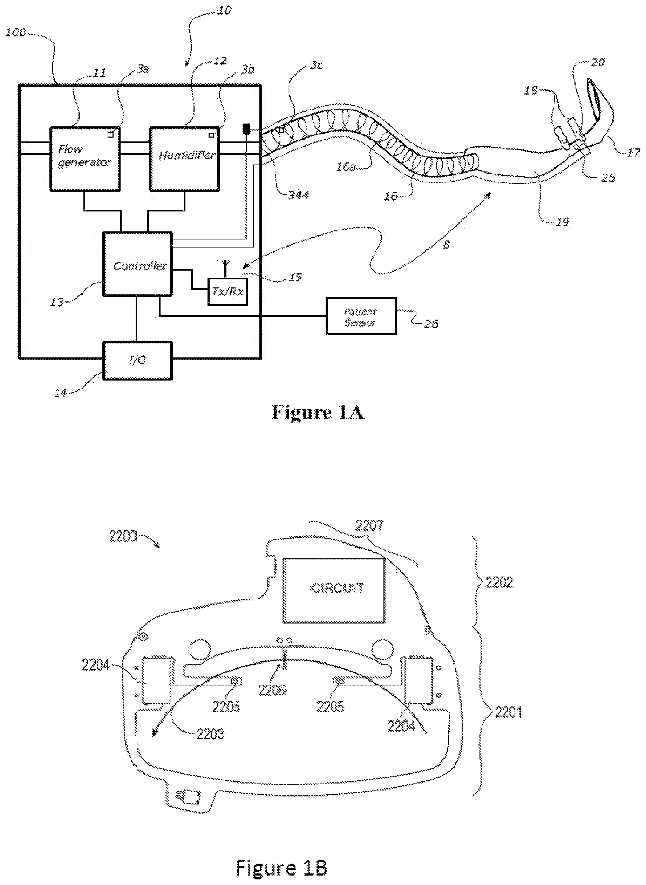

A high flow respiratory support apparatus 10 is shown in Figure 1A. The respiratory support apparatus 10 can comprise a main housing 100 that contains a flow generator 11 in the form of a motor/impeller arrangement (for example, a blower), an optional humidifier 12, a controller 13, and a user interface 14 (comprising, for example, a display and input device(s) such as button(s), a touch screen, or the like). The controller 13 can be configured or programmed to control the operation of the apparatus. For example, the controller can control components of the apparatus, including but not limited to: operating the flow generator 11 to create a flow of gas (gases flow) for delivery to a patient, operating the humidifier 12 (if present) to humidify and/or heat the generated gases flow, controlling a flow of oxygen into the flow generator blower, receiving user input from the user interface 14 for reconfiguration and/or user-defined operation of the apparatus 10, and outputting information (for example on the display) to the user. The user can be a patient, healthcare professional, or anyone else interested in using the apparatus. As used herein, a "gases flow" can refer to any flow of gases that may be used in the breathing assistance or respiratory device, such as a flow of ambient air, a flow comprising substantially 100% oxygen, a flow comprising some combination of ambient air and oxygen, and/or the like.

A patient breathing conduit 16 is coupled at one end to a gases flow outlet 21 in the housing 100 of the respiratory support apparatus 10. The patient breathing conduit 16 is coupled at another end to a patient interface 17 such as a non-sealed nasal cannula with a manifold 19 and nasal prongs 18. Additionally, or alternatively, the patient breathing conduit 16 can be coupled to a face mask, a nasal mask, a nasal pillows mask, an endotracheal tube, a tracheostomy interface, and/or the like. Preferably in the respiratory support apparatus 10 the patient interface 17 is an unsealed interface in order to provide high flow respiratory support to a patient. The gases flow that is generated by the respiratory support apparatus 10 may be humidified and delivered to the patient via the patient conduit 16 through the cannula 17. The patient conduit 16 can have a heater wire 16a to heat gases flow passing through to the patient. The heater wire 16a can be under the control of the controller 13. The patient conduit 16 and/or patient interface 17 can be considered part of the respiratory support apparatus 10, or peripheral to the respiratory support apparatus. The respiratory support apparatus 10, breathing conduit 16, and patient interface 17 together can form a respiratory support system.

The apparatus 10 may comprise:
a. A housing with the humidifier and flow generator disposed in the housing.
b. An elbow that fluidly couples the outlet of the humidifier to the outlet of the apparatus. The elbow is removable such that the elbow can be disinfected or replaced to reduce the chances of infecting a patient.

The controller 13 can control the flow generator 11 to generate a gases flow of the desired flow rate. The controller 13 can also control a supplemental oxygen inlet to allow for delivery of supplemental oxygen, the humidifier 12 (if present) can humidify the gases flow and/or heat the gases flow to an appropriate level, and/or the like. The gases flow is directed out through the patient conduit 16 and cannula 17 to the patient. The controller 13 can also control a heating element in the humidifier 12 and/or the heating element 16a in the patient conduit 16 to heat the gas to a desired temperature for a desired level of therapy and/or level of comfort for the patient. The controller 13 can be programmed with or can determine a suitable target temperature of the gases flow. The temperature can comprise a temperature of the gases flow, or a dew-point of the gases flow.

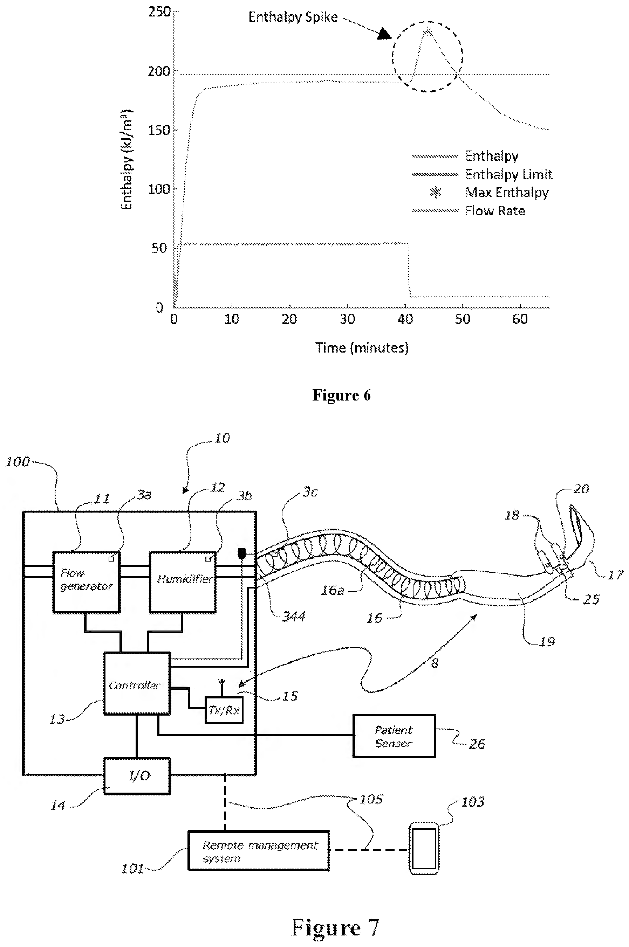

The oxygen inlet port 28 can include a valve through which a pressurized gas may enter the flow generator or blower. The valve can control a flow of oxygen into the flow generator blower. The valve can be any type of valve, including a proportional valve or a binary valve. The source of oxygen can be an oxygen tank or a hospital oxygen supply. Medical grade oxygen is typically between 95% and 100% purity. Oxygen sources of lower purity can also be used. Examples of valve modules and filters are disclosed in U.S. Provisional Application No. 62/409,543, titled "Valve Modules and Filter", filed on October 18, 2016, and U.S. Provisional Application No. 62/488,841, titled "Valve Modules and Filter", filed on April 23, 2017, which are hereby incorporated by reference in their entireties. Valve modules and filters are discussed in further detail below with relation to Figure 6.

The respiratory support apparatus 10 can, in some examples, measure and control the oxygen content of the gas being delivered to the patient, and therefore the oxygen content of the gas inspired by the patient. During high flow respiratory support or treatment (i.e. high flow therapy), the high flow rate of gas delivered meets or exceeds the peak inspiratory demand of the patient. This means that the volume of gas delivered by the device to the patient during inspiration meets, or is in excess of, the volume of gas inspired by the patient during inspiration. High flow respiratory support (i.e. high flow therapy) therefore helps to prevent entrainment of ambient air when the patient breathes in, as well as flushing the patient's airways of expired gas. So long as the flow rate of delivered gas meets or exceeds peak inspiratory demand of the patient, entrainment of ambient air prevented, and the composition of the gas delivered by the device is substantially the same as the composition of the gas the patient breathes in. As such, the oxygen fraction measured in the device, known as the fraction of delivered oxygen (FdO₂), would be substantially the same as the oxygen fraction of the gas that the user inspires, known as the fraction of inspired oxygen (FiO₂). Therefore, the terms FdO₂ and FiO₂ may be seen as equivalent in this context. Note that in other examples, apparatus 10 may not include any oxygen control, and may provide high flow to a patient without any supplementary oxygen being provided, i.e. the inspiratory gases flow to the patient is the flow generated from ambient air through the flow generator inlet, which may be optionally humidified.

Operation sensors 3a, 3b, 3c, such as flow, temperature, humidity, and/or pressure sensors can be placed in various locations in the respiratory support apparatus 10. Additional sensors (for example, sensors 20, 25) may be placed in various locations on the patient conduit 16 and/or cannula 17 (for example, there may be a temperature sensor 29 at or near the end of the inspiratory tube, or at the patient interface). Output from the sensors can be received by the controller 13, to assist the controller in operating the respiratory support apparatus 10 in a manner that provides suitable therapy. In some configurations, providing suitable therapy includes meeting a patient's peak inspiratory demand. The apparatus 10 may have a transmitter and/or receiver 15 to enable the controller 13 to receive signals 8 from the sensors and/or to control the various components of the respiratory support apparatus 10, including but not limited to the flow generator 11, humidifier 12, and heater wire 16a, or accessories or peripherals associated with the respiratory support apparatus 10. Additionally, or alternatively, the transmitter and/or receiver 15 may deliver data to a remote server or enable remote control of the apparatus 10.

Oxygen may be measured by placing one or more gas composition sensors (such as an ultrasonic transducer system, also referred to as an ultrasonic sensor system) downstream from the point in the flow path that the oxygen and ambient air finish mixing. The measurement can be taken within the device, the delivery conduit, the patient interface, or at any other suitable location. The particular sensor or sensors used can be varied depending on control requirements. In one example the ultrasonic transducer system is positioned within the gases flow path, within the housing of the respiratory support apparatus.

The respiratory support apparatus 10 may, in some examples, include a patient sensor 26, such as a pulse oximeter or a patient monitoring system, to measure one or more physiological parameters of the patient, such as a patient's blood oxygen saturation (SpO2), heart rate, respiratory rate, or perfusion index. The patient sensor 26 may also provide a measure of signal quality. The sensor 26 can communicate with the controller 13 through a wired connection or by communication through a wireless transmitter on the sensor 26. The sensor 26 may be a disposable adhesive sensor designed to be connected to a patient's finger. The sensor 26 may be a non-disposable sensor. Sensors, designed for different age groups and to be connected to different locations on the patient, are available and can be used with the respiratory support apparatus.

Where the patient sensor 26 is a pulse oximeter, this is attached to the patient, typically at their finger, although other places such as an earlobe are also an option. The pulse oximeter is connected to a processor in the apparatus 10, for example to controller 13, and constantly provides signals indicative of the patient's blood oxygen saturation.

The patient sensor 26 can be a hot-swappable device, which can be attached or interchanged during operation of the respiratory support apparatus 10. For example, the patient sensor 26 may connect to the respiratory support apparatus 10 using a USB interface or using wireless communication protocols (such as, for example, near field communication, WiFi or Bluetooth^{®}). When the patient sensor 26 is disconnected during operation, the respiratory support apparatus 10 may continue to operate in its previous state of operation for a defined time period. The patient sensor 26 may be a bedside monitoring system or other patient monitoring system that communicates with the respiratory support apparatus 10 through a physical or wireless interface.

High flow respiratory support, as discussed herein, is intended to be given its typical ordinary meaning as understood by a person of skill in the art, which generally refers to a respiratory assistance system delivering a targeted flow of humidified respiratory gases via an intentionally unsealed patient interface with flow rates generally intended to meet or exceed the inspiratory flow of a patient. Typical patient interfaces include, but are not limited to, nasal or tracheal patient interfaces. Typical flow rates for adults often range from, but are not limited to, about fifteen litres per minute (L/min) to about seventy litres per minute or greater. Typical flow rates for paediatric patients (such as neonates, infants and children) often range from, but are not limited to, about one litre per minute per kilogram of patient weight to about three litres per minute per kilogram of patient weight or greater. High flow respiratory support (i.e. high flow therapy) can also optionally include gas mixture compositions including supplemental oxygen and/or administration of therapeutic medicaments. High flow respiratory support is often referred to as nasal high flow (NHF), humidified high flow nasal cannula (HHFNC), high flow nasal oxygen (HFNO), high flow therapy (HFT), or tracheal high flow (THF), among other common names. The flow rates used to achieve "high flow" (i.e. high flow respiratory support) may be any of the flow rates listed below. For example, in some configurations, for an adult patient 'high flow respiratory support' may refer to the delivery of gases to a patient at a flow rate of greater than or equal to about 10 litres per minute (10 L/min), such as between about 10 L/min and about 100 L/min, or between about 15 L/min and about 95 L/min, or between about 20 L/min and about 90 L/min, or between 25 L/min and 75 L/min, or between about 25 L/min and about 85 L/min, or between about 30 L/min and about 80 L/min, or between about 35 L/min and about 75 L/min, or between about 40 L/min and about 70 L/min, or between about 45 L/min and about 65 L/min, or between about 50 L/min and about 60 L/min. In some configurations, for a neonatal, infant, or child patient 'high flow respiratory support' (i.e. high flow therapy) may refer to the delivery of gases to a patient at a flow rate of greater than 1 L/min, such as between about 1 L/min and about 25 L/min, or between about 2 L/min and about 25 L/min, or between about 2 L/min and about 5 L/min, or between about 5 L/min and about 25 L/min, or between about 5 L/min and about 10 L/min, or between about 10 L/min and about 25 L/min, or between about 10 L/min and about 20 L/min, or between about 10 L/min and 15 L/min, or between about 20 L/min and 25 L/min. A high flow respiratory support apparatus with an adult patient, a neonatal, infant, or child patient, may deliver gases to the patient at a flow rate of between about 1 L/min and about 100 L/min, or at a flow rate in any of the sub-ranges outlined above. The respiratory support apparatus 10 can deliver any concentration of oxygen (e.g. FdO₂), up to 100%, at any flowrate between about 1 L/min and about 100 L/min. In some configurations, any of the flowrates can be in combination with oxygen concentrations (FdO₂s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 L/min and 75 L/min in combination with an oxygen concentration (FdO₂) of about 20%-30%, 21%-30%, 21%-40%, 30%-40%, 40%-50%, 50%-60%, 60%-70%, 70%-80%, 80%-90%, and 90%-100%. In some configurations, the respiratory support apparatus 10 may include safety thresholds that prevent a user from delivering to much oxygen to the patient.

High flow respiratory support may be administered to the nares of a user and/or orally, or via a tracheostomy interface. High flow respiratory support may deliver gases to a user at a flow rate at or exceeding the intended user's peak inspiratory flow requirements. The high flow respiratory support may generate a flushing effect in the nasopharynx such that the anatomical dead space of the upper airways is flushed by the high incoming gases flow. This can create a reservoir of fresh gas available for each breath, while minimizing re-breathing of nitrogen and carbon dioxide. Meeting inspiratory demand and flushing the airways is additionally important when trying to control the patient's FdO₂. High flow respiratory support can be delivered with a non-sealing patient interface such as, for example, a nasal cannula. The nasal cannula may be configured to deliver breathing gases to the nares of a user at a flow rate exceeding the intended user's peak inspiratory flow requirements.

The term "non-sealing patient interface" as used herein can refer to an interface providing a pneumatic link between an airway of a patient and a gases flow source (such as from flow generator 11) that does not completely occlude the airway of the patient. A non-sealed pneumatic link can comprise an occlusion of less than about 95% of the airway of the patient. The non-sealed pneumatic link can comprise an occlusion of less than about 90% of the airway of the patient. The non-sealed pneumatic link can comprise an occlusion of between about 40% and about 80% of the airway of the patient. The airway can include one or more of a nare or mouth of the patient. For a nasal cannula the airway is through the nares.

The flow generator or blower 11 can include an ambient air inlet port 27 through which ambient room air can be drawn into the blower.

The respiratory support apparatus 10 may also include an oxygen inlet port 28 leading to a valve through which a pressurized gas may enter the flow generator or blower 11. The valve can control a flow of oxygen into the flow generator blower 11. The valve can be any type of valve, including a proportional valve or a binary valve.

The blower can operate at a motor speed of greater than about 1,000 RPM and less than about 30,000 RPM, greater than about 2,000 RPM and less than about 21,000 RPM, greater than about 4,000 RPM and less than about 19,000 RPM, or between any of the foregoing values. Operation of the blower can mix the gases entering the blower through the inlet ports. Using the blower as the mixer can decrease the pressure drop that would otherwise occur in a system with a separate mixer, such as a static mixer comprising baffles, because mixing requires energy. Having a static mixer can also increase the volume of the gas flow path between the valve and the gases composition sensor, which can further increase the delay between when the valve current is changed and when a corresponding change in oxygen concentration is measured.

Based on user inputs and the therapy supplied by the specific device, the controller can determine a target value for an output parameter of the blower (e.g. a flow rate). The controller can receive measurements of the output parameter and adjust the speed of the blower based on the difference between the target value and the measured value.

The target output parameter may be flow rate. The target flow rate may be a constant value. The target flow rate may be a value that fluctuates. In some configurations, the controller can control a blower motor speed based on a target flow rate, and additionally increase or decrease the motor speed based on a patient's breathing cycle. The target flow rate does not necessarily change, but the controller causes the motor speed to fluctuate in order to add oscillations to the instantaneous flow rate, so that it is synchronized with the patient's breathing. Such a system is described in International Application No. PCT/NZ2017/050063, titled "Flow Path Sensing for Flow Therapy Apparatus", filed on May 17, 2017.

With additional reference to Figure 1B, a sensing circuit board 2200 is shown that can be implemented in the respiratory support apparatus 10. The sensing circuit board 2200 can be positioned in a sensor chamber such that the sensing circuit board 2200 is at least partially immersed in the flow of gases. The flow of gases may exit the blower 11 through a conduit and enter a flow path in the sensor chamber. At least some of the sensors on the sensing circuit board 2200 can be positioned within the flow of gases to measure gas properties within the flow. After passing through the flow path in the sensor chamber, the gases can exit to the humidifier 12 described above.

The sensing circuit board 2200 can be a printed sensing circuit board (PCB). Alternatively, the circuit on the board 2200 can be built with electrical wires connecting the electronic components instead of being printed on a circuit board. At least a portion of the sensing circuit board 2200 can be mounted outside of a flow of gases. The flow of gases can be generated by the flow generator 11 described above. The sensing circuit board 2200 can comprise ultrasonic transducers 2204. The sensing circuit board 2200 can comprise one or more thermistors 2205. The thermistors 2205 can be configured to measure a temperature of the gases flow. The sensing circuit board 2200 can comprise a thermistor flow rate sensor 2206. The sensing circuit board 2200 can comprise other types of sensors, such as humidity sensors (including humidity-only sensors to be used with a separate temperature sensor and combined humidity and temperature sensors), sensors for measuring barometric pressure, sensors for measuring differential pressure, and/or sensors for measuring gauge pressure. The thermistor flow rate sensor 2206 can comprise a hot wire anemometer, such as a platinum wire, and/or a thermistor, such as a negative temperature coefficient (NTC) or positive temperature coefficient (PTC) thermistor. Other nonlimiting examples of the heated temperature sensing element include glass or epoxy-encapsulated or non-encapsulated thermistors. The thermistor flow rate sensor 2206 can be configured to measure the flow rate of the gases by being supplied with a constant power, by being maintained at a constant sensor temperature, or by being maintained at a temperature that is different to the temperature of the flow of gases by a constant amount.

The sensing circuit board 2200 can comprise a first portion 2201 and a second portion 2202. The first portion 2201 can be positioned to be within the flow path of the gases, whereas the second portion 2202 can be positioned to be outside the flow path of the gases. The direction of the flow of gases is indicated in Figure 1B by the arrow 2203. The direction of the flow of gases can be a straight line, or curved, as shown in Figure 1B.

Positioning one or more of the thermistors 2205 and/or the thermistor flow rate sensor 2206 downstream of the combined blower and mixer can take into account heat supplied to the gases flow from the blower. Immersing the temperature-based flow rate sensors in the flow path can provide a better representation of the characteristics of the gases flow, as this immersion results in the sensors being subject to the conditions within the gases flow.

The sensing circuit board 2200 can comprise ultrasonic transducers, transceivers, or sensors which are configured to measure properties of the gases flow, such as composition. Any suitable transducer, transceiver, or sensor may be mounted to the sensing circuit board 2200 as will be appreciated. For example, the sensing circuit board can include an ultrasonic transducer system (also referred to as an ultrasonic sensor system) that employs ultrasonic or acoustic waves for determining gas concentrations.

The controller 13 may control the conduit heater 16c, and/or the humidifier 12 based on feedback from one or more of the patient end sensors 26, such as a temperature sensor and/or humidity sensor, at a patient end of the conduit 9. The patient end sensor(s) 26 may be located at the patient end of the conduit, for example in a cuff or connector at the end of the conduit. The patient end sensor(s) may be located in the patient interface 17. The controller 13 may be configured to determine a temperature drop from the end of the inspiratory conduit 9 to the patient, for example a 3 °C drop, and to control the conduit heater 16c and/or humidifier 12 using feedback control, that is, using closed loop control. Such a control methodology is disclosed in our earlier patent US8453641.

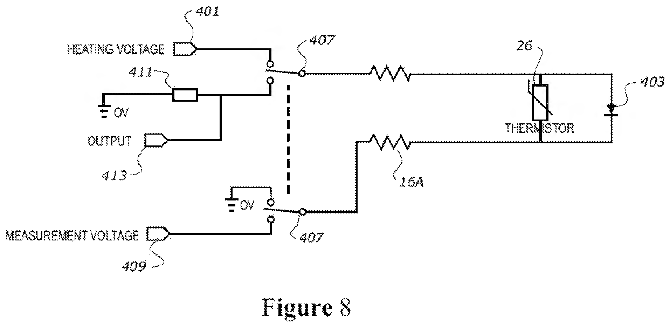

With reference to Figure 8, a circuit 401 is provided that may be utilised for carrying out a method of measuring temperature using a patient end sensor 26, for control the temperature and/or dewpoint of the inspiratory gases flow during operation of an apparatus in accordance with this disclosure, and in particular during a high-temperature mode and/or a cool-down mode.

When a DC heating voltage 401 is applied to the heater wire 16A, a diode 403 conducts and current flows through the heater wire 16A and the heater wire 16A functions as normal and provides heating to the delivery tube 9. When the heating voltage 401 is switched off using switch 407, a measurement voltage 409, which has opposite polarity to the heating voltage 401 5 is applied to the heater wire 16A. In this case, the current in the heater wire 16A does not flow through the diode 403 but flows through the patient end sensor 26, which may be a thermistor, and through a reference resistor 411. The voltage across the reference resistor 411 can then be measured at the output 413 and the temperature of the gases determined. The voltage measurement 413 across the reference resistor 411, is converted to a temperature using a look up table or an equation to calculate a value for temperature.

More generally, the thermistor 26 may be replaced by an impedance (for example, a resistor and a capacitive sensor) for pressure or humidity measurement. Either the impedance can be measured by measuring the voltage across the reference resistor 411 or the rise-time could be determined by looking at the voltage across the reference resistor 411 in time.

Part of the circuit could be included in the delivery conduit 9 and in particular the diode 403 and thermistor 26 (in parallel with one another) are preferably placed in series with the heater wire 16A at a point in the heater wire 16A at or near the end of the delivery tube 9 nearest the patient. For example they may be interconnected on a printed circuit board, overmolded with plastic for sealing and mounted in the gases stream through the delivery conduit 9. Furthermore, the circuit may be formed by interconnected parts in a housing, for example, a plastic housing, that protrudes from the plastic wall of the delivery tube into the gases flow through the conduit, in order to measure that gases properties. All other parts of the circuit including the reference resistor 411 and the switching circuitry 407 would be included in the control circuitry of the apparatus 100.

The thermistor's 26 value can be chosen to have different resistance curves with known properties at ambient temperature. The choice of a particular thermistor value for use with the circuit allows identification by the controller 13 and matching of that thermistor value with a specific conduit or tubing 9 and/or patient interface 17. Different thermistor values can be matched with a particular and appropriate conduit and/or patient interface types and upon connection of the conduit 9 to apparatus 100, the controller 13 can identify that thermistor 26 and apply the appropriate control strategy to the heating of the conduit 9.

Positioning sensors in the flow path for example, instead of outside the flow path, allows the transducers 2204 to both operate within a smaller temperature range relative to one another, or both substantially at one temperature (namely, the temperature of the gases flow). As the transducers are sensitive to temperature, having them at a substantially homogenous temperature increases accuracy. Further, positioning sensors along the flow path allows for measurements and calculations that account for the influence of the gas velocity so that the effect of gas velocity can be removed from the sensor measurement.

In some configurations, the respiratory support apparatus may also be provided with a humidity sensor that is located in the flow path and which is configured to generate a humidity signal indicative of the humidity of the gases stream flowing through the sensor assembly. In such configurations, the gas composition may be determined by the sensed speed of sound, and the sensed temperature and/or sensed humidity. The humidity sensor may be a relative humidity sensor or an absolute humidity sensor. In some embodiments, the gas composition may be determined based on the sensed speed of sound and the sensed humidity, without the need for a temperature sensor.

Some examples of respiratory support apparatuses are disclosed in International Application No. PCT/NZ2016/050193, titled "Flow Path Sensing for Flow Therapy Apparatus", filed on December 2, 2016, and International Application No. PCT/IB2016/053761, titled "Breathing Assistance Apparatus", filed on June 24, 2016, and US patent US8453641 filed 19 August 2005, each of which are hereby incorporated by reference in their entireties. Examples of configurations of respiratory support apparatuses that can be used with aspects of the present disclosure are discussed in further detail below.

The various configurations described are exemplary configurations only. Any one or more features from any of the configurations may be used in combination with any one or more features from any of the other configurations.

The apparatus 10 comprises a controller 13. The controller 13 may comprise one or more electronic data processors, operative according to a combination of hardware, firmware, and software. The controller 13 may comprise one or more memory units. The controller 13 may comprise one or more sub-controllers. A separate controller may be provided to control each component of the apparatus 10, for example a controller for the flow generator, a controller for the humidifier, etc. Use of the term 'controller' may refer to any of these configurations.

The apparatus 10 further comprises an input/output device 14 in the form of one or more user interfaces. This input/output device 14 is configured to allow a user to input one or more control signals to the controller 13. Conveniently the input/output device 14 may be configured to provide one or more graphic user interfaces to the user, allowing the user to view and control operating parameters of the apparatus 10. The input/output device 14 may comprise a touch screen interface.

The controller 13 is configured to control the apparatus 10 to provide a high flow of gases to the patient as discussed above. In particular, the controller 13 controls the flow generator to generate a gases flow at a desired flow rate. The controller 13 also controls the humidifier to heat and humidify the gases flow prior to delivery to the patient. The controller may control the humidifier to heat the gases flow to a temperature set point. The temperature set point may be determined on the basis of a desired gases flow dew-point. The dew-point is function of both the gases flow temperature and the gases flow humidity. Control of both of these parameters enables control of the dew-point. The controller may additionally or alternatively control the humidifier to humidify the gases flow to a measured or calculated humidity (in which case, for a given gases flow temperature, the dew-point will change with changing humidity).

The controller 13 is configured to control the apparatus 10 in a normal mode and a high-temperature mode. In both modes, the apparatus 10 may be configured to control both the temperature and the humidity of the gases flow, thus ensuring the apparatus can deliver the gases flow at a desired dew-point (typically known as the set point). Alteration of the set point for the dew-point by the user, or automatically by the controller, controls the apparatus to vary the temperature and/or humidity of the gases flow to deliver the gases flow at the dew-point set point. These modes can be manually selected by the user, using the user interface 14. In both modes, a high flow of gases is generated and delivered to the patient. In normal mode the flow rates of the gases flow delivered to the patient are as per the normal high flow rates set out above in paragraph [00135] for example. In normal mode, the dew-point of the gases flow delivered to the patient is typically less than 39 °C, and more typically in the range of 31 to 37 °C. In high-temperature mode the dew-point of the gases flow delivered to the patient is higher than in normal mode, and in particular is in the range of 43 to 50 °C. The temperature in high-temperature mode may be in the range of 41 to 50 °C. The temperature in high-temperature mode may be in the range of 41 to 49 °C. The temperature in high-temperature mode may be in the range of 43 to 47 °C. In other words, the gases flow temperature set point, to which the apparatus attempts to deliver the gases flow, is higher in high-temperature mode than in normal mode. The apparatus 10 is configured, to increase the dew-point of the gases flow between the two modes, so that gases are delivered with a higher dew-point in high-temperature mode.

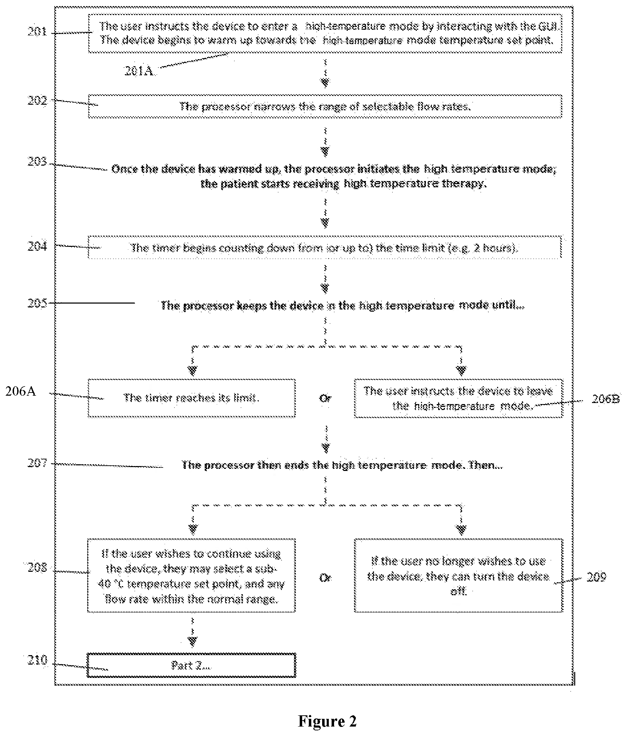

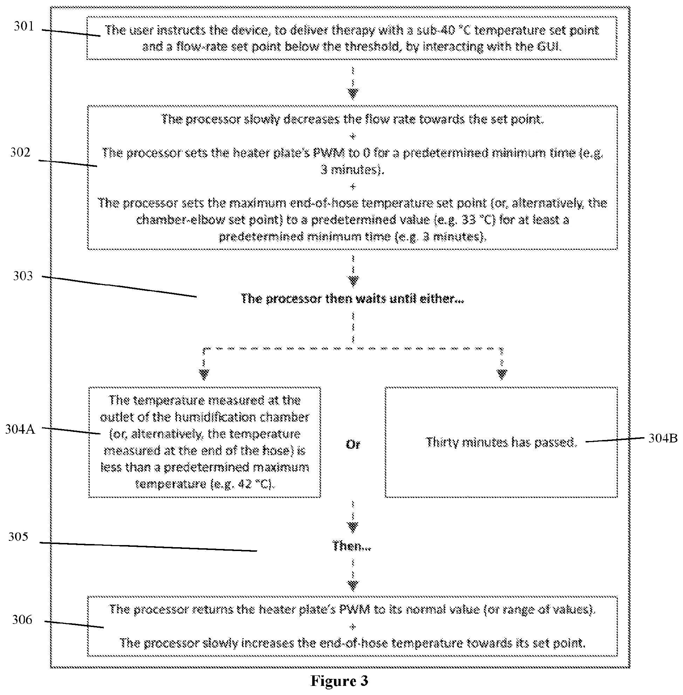

In providing the high-temperature mode, the apparatus 10 is operative according to one or more safety algorithms that ensure that the properties of the gases flow delivered to the patient, are safe both during and after high-temperature mode. These safety algorithms control the gases flow to ensure that the thermal energy and/or enthalpy in the gases flow is below safe levels. The safety algorithms may therefore be operative according to parameters including gases temperature, gases dew-point, gases flow rate, and/or duration of high-temperature mode and a subsequent cool-down mode. A method of controlling an apparatus 10 during a high temperature mode is described below with reference to Figure 2. A method of controlling an apparatus 10 according to a subsequent cool-down mode is described below with reference to Figure 3.

Referring initially to Figure 2, when high-temperature mode is selected by the user, the controller 13 receives an instruction 201 from the user to enter the high-temperature mode.

The apparatus 10 is controlled to warm up at step 201A. In particular, the humidifier heater plate, and/or the conduit heater 16c are warmed up. As the apparatus begins to warm up towards the higher temperature set point of the high-temperature mode, the controller 13 begins at step 203 to enact a high-temperature control algorithm, or at least a first part of such an algorithm, (referenced 'Part 1' in the figures), to help keep the patient safe during the high-temperature mode session, that is, safe from excessive enthalpy/dewpoint during that therapy session. Part I is in accordance with the process shown in Figure 2.

Once the apparatus 10 has warmed up at step 203, high temperature therapy is provided to the patient, that is, an inspiratory gases flow at high temperature. Once high temperature therapy has begun, a timer is initiated at step 204, to provide a timing signal that is used by controller 13 to limit the duration of high-temperature mode to a predetermined, or user programmed duration. At step 204, the apparatus 10 continues to operate in high-temperature mode until either the time duration has reached its limit as per step 206A, or the user has otherwise instructed the apparatus 10 to terminate high-temperature mode in step 206B. The apparatus 10 then terminates high-temperature mode at step 207.

If the user wishes to continue using the apparatus 10, they may do so at step 208. Alternatively, they may terminate all therapy at step 209. At step 208, the user may select one or more new operating parameters with which to continue therapy for example temperature set-point or flow rate. As will be described below with reference to Figure 3, the apparatus 10 is then controlled at step 210 according to a cool-down mode in which one or more parameters of the gases flow to the patient are controlled such that the gases flow remains safe for the patient.

Referring now to Figure 3, the apparatus 10 is ow operative according to a cool-down mode, following termination of the high-temperature mode. At step 301 of the cool-down mode, the apparatus 10 receives an instruction from the user regarding a desired parameter of the gases flow, for example temperature set-point and/or flow rate. Such instruction is received via the user interface 14, or via a remote management system 101 as will be described further below.

At step 302, the apparatus is controlled according to one or more initial safety processes. These safety processes include any one or more of: slowly decreasing the flow rate towards a cool-down mode flow rate set point, setting the humidifier heater plate PWM to zero for a predetermined period of time, and setting a maximum end of hopes temperature to a predetermined value that is considered safe for the user. The end of hose temperature can be measured by patient end sensor 26, and feedback control used by the controller accordingly.

Once the one or more safety processes have been set, the controller waits at 303 until either a measured temperature is less than a predetermined maximum safe temperature as per step 304A, or a predetermined period of time as passed as per step 304B. Step 304A could use a temperature measured anywhere downstream of the humidifier, for example, at the humidifier outlet, or at the end of hose using the patient end sensor 26. The predetermined period of time may be 30 minutes in one example.

At step 305 the apparatus checks that one of steps 304A or 304B is satisfied, and if so, moves to step 306 which terminates the cool-down mode, and transitions the apparatus to normal mode. During this transition the apparatus returns the humidifier heater plate's PWM to its normal value or range of values, and slowly increases the end of hose temperature, as measured by patient end sensor 26, towards the normal set point.

When enacting the first part of this algorithm, namely the high-temperature mode, the controller reduces the peak flow rate of the gases flow, from the higher peak flow rate used in normal mode. The controller 13 may also narrow the range of selectable flow rates (i.e. the range of flow rates that can be selected by the user), when in high-temperature mode. For example, the range of selectable flow rates may change from 10 to 70 L/min down to a narrower range of 30 to 40 L/min, 30 to 70 L/min, or 10 to 40 L/min. The controller 13 may reduce the range of flow rates to a predetermined amount below the range of flow rates used in normal mode. For example, the range of flow rates may be reduced by 15L/min, or 10L/min. The controller 13 may increase or decrease the upper and/or lower ends of the flow rate range. It may be appropriate to increase the upper end of the flow rate range, such that the residual energy in the system does not cause the temperature of the gases flow to spike. Likewise it may be appropriate to increase the lower end of the flow range.

Once the apparatus 10 has warmed up, the controller 13 initiates the high-temperature mode as a high-temperature session, in which the gases flow is delivered at the high temperatures indicated above, and simultaneously begins a session timer. The user and/or the patient can control the controller 13 to initiate high-temperature mode. As noted above, in high-temperature mode, the gases flow is controlled to a temperature range in which the peak temperature is higher than the peak temperature of the temperature range used in normal mode. The controller 13 ends the high-temperature session when, either, the session timer reaches its safety limit, or the user instructs the apparatus 10 to terminate the high-temperature mode. At this point, the controller 13 has reached the end of the first part of the high-temperature control algorithm (labelled 'Part 1' in the figures). The safety limit is a set duration determined with reference to the timer, and will typically be 120 minutes or less. The user can set a duration that is less than the maximum that the apparatus will allow in a high-temperature mode, for example 30 or 60 minutes. The set duration could be determined with reference to a counter (a predetermined number of counts leading to termination of the high-temperature mode). The timer or counter could be generated by an algorithm on the controller 13, or the controller 13 could be configured to receive a timer or counter signal from another controller (for example, a dedicated timer/counter controller), or from a remote device (for example remote device 103 via remote management system 01, as described below). When the apparatus 10 is warming up there may be a message provided on the screen of the user interface 14, or a message could be sent to the mobile device of the patient. Once warm up mode is complete a separate message may be presented on the user interface 14 or sent to the patient.

If the user wishes to continue using the apparatus in normal mode (for example with a sub-40 °C dew-point set point and a flow-rate set point below a predetermined threshold), the controller begins to enact the second part of the high-temperature mode algorithm (labelled 'Part 2' in the figures) which is a cool-down mode configured to help keep the patient safe while the apparatus cools. Note that the threshold may be predetermined (e.g. factory-set), or a function of the flow rate that was used during high-temperature mode. Either way, the threshold represents the highest flow rate that the device can quickly transition to, after high-temperature mode, without generating a spike in the dew-point (and/or enthalpy) of the gases flow. Such a spike can occur if the apparatus transitions too quickly from high-temperature mode to normal mode (with a low flow rate) - due to the residual energy in the apparatus.

In cool-down mode, the controller slowly alters the gases flow rate towards its new set point and reduces the electrical power supplied to the humidifier heater plate. The cool-down mode is in accordance with the process shown in Figure 3. The controller can reduce the electrical power supplied to the heater plate, for example by setting the heater plate's PWM to zero for at least a predetermined time. The power supplied to the heater plate could alternatively or additionally be controlled by controlling the heater plate duty cycle, or the voltage and/or current supplied to the heater plate. In cool-down mode, the controller 13 also sets the maximum gases flow temperature set point, at a specific point downstream of the humidification, to a predetermined value for at least a predetermined time.

The controller 13 is thus operative to control the rate of change of the flow rate of the gases flow as that flow rate transitions from the flow rate used in high-temperature mode to the flow rate that is to be subsequently used in normal mode. This helps ensure that the flow rate does not drop too quickly whilst there is still thermal energy in the apparatus (that thermal energy having been generated during the just terminated high-temperature mode). As can be seen in Figure 6, and explained further below, if the flow rate drops to too low a value, there would be relatively little gas passing through the system but a relatively high residual thermal energy. This could lead to the gases flow becoming excessively heated during cool-down, increasing the gases dew-point/enthalpy to a level that is harmful to the patient.

The controller 13 is also operative to at least reduce the electrical energy supplied to the humidifier heater during the cool-down mode, and may reduce that electrical energy to zero. This ensures that the heater, during cool-down, is not adding thermal energy to the thermal system, further helping to ensure the gases temperature remains below safe levels. If the apparatus 10 comprises a conduit heater 16c, in the gases delivery conduit 9 to the patient, the electrical energy supplied to such a conduit heater 16c can also be controlled, if required.

Further, the apparatus 10 is operative according to a new dew-point set point for the gases flow, which set point is preferably in the range of temperatures of normal mode. This new dew-point set-point may be set by the user. This helps to ensure that the gases flow temperature does not exceed a safe level during cool-down. The apparatus 10 is thus configured to manage the potentially high thermal energy/enthalpy residual in the components of the apparatus immediately after high-temperature mode, thus minimizing the likelihood of the patient being exposed to a gases flow having a dew-point and/or enthalpy above a safe level.

Once the temperature measured downstream of the humidifier, for example at the patient interface, falls below a threshold value, and/or a predetermined time following the humidifier heater PWM being reduced or set to zero elapses, the controller returns the humidifier heater PWM to its normal range of values (as used during normal mode) and slowly increases the end-of-hose gases flow temperature towards its new temperature set point (as used during normal mode). At this point, the controller has reached the end of the second part of the high-temperature algorithm.

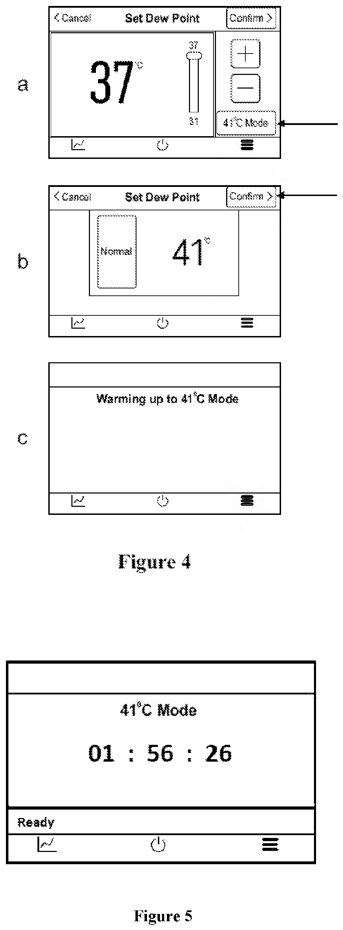

Example screens of the graphic user interface of user interface 14 for details A, B, and C of Figure 2 are shown in Figure 4.

In one example, the user can initiate a version of a high-temperature mode (in this example, '41 °C Mode') by first navigating to the "Set Dew-point" screen on the high flow device's graphic user interface (GUI), as per Figure 4A. From this screen the user presses the virtual "41 °C Mode" button (this button may display a different dew-point if the specified high-temperature mode dew-point is different). This button prompts the GUI to display a screen which informs the user that they have selected the high-temperature 41 °C Mode (Figure 4B). This screen has a virtual "Confirm" button. It is noted that the virtual buttons of the GUI, could be replaced or supplemented by electromechanical buttons adjacent a non-touchscreen display.

When the user presses the virtual "Confirm" button, the safety algorithm described above immediately narrows the range of selectable flow rates by changing one, or both, of the minimum and maximum flow rate range limits (for example the range of selectable flow rates may change from 10 to 70 L/min to 30 to 40 L/min, 30 to 70 L/min, or 10 to 40 L/min). When this button is pressed, the safety algorithm also begins increasing the dew-point of the gases flow towards the set point used in high-temperature mode, for example 41 °C. While the apparatus is increasing the dew-point, the GUI displays a screen that informs the user that the apparatus is "Warming up to 41 °C Mode" (Figure 4C).

Once the apparatus has increased the set point to 41 °C, the high-temperature mode (in this example '41 °C Mode') begins. As soon as the apparatus enters the 41 °C Mode, the safety algorithm begins a timer that counts down from (or up to) a set duration of for example 2 hours. This set duration defines a maximum duration in which the apparatus can operate in high-temperature mode. The user can choose to have this timer displayed on the GUI during 41 °C mode, see for example Figure 5. The user can manually exit high-temperature mode before the maximum duration has been reached. If the user does not manually exit, once the maximum duration has been reached, the apparatus will automatically terminate high-temperature mode and enter cool-down mode.

With reference to Figure 6, to understand the importance of the cool-down mode, consider a use case in which the user selects a flow rate below 30 L/min (but above 0 L/min) immediately after the apparatus has terminated high-temperature mode. If the flow rate immediately dropped before the humidifier chamber temperature had had time to decrease below its high-temperature mode set point, then the amount of heat absorbed by each unit volume of breathing gases would increase, resulting in a sharp increase (spike) in the enthalpy (and/or dew-point) of the gases flow at the patient interface. This spike would occur even if the electrical energy supplied to the humidifier heater was switched to 0% as soon as the flow rate dropped, due to the time taken for the water to cool. Eventually the water would cool, and the enthalpy would decrease, however, the enthalpy spike could be enough to exceed the safe enthalpy limit (as in the example shown in Figure 6).

In accordance with this disclosure, we also provide a respiratory support apparatus 10 in which the flow generator is omitted. The apparatus 10 still comprises a humidifier 12, and the other components described above and as shown in Figure 1A. Such a respiratory support apparatus comprises one or more gas(es) inlets configured to receive one or more gases from one or more external gas supplies. The external gas supply may comprise, for example, a hospital wall oxygen supply, or a supply of gas from a gas cannister or tank.

In such a respiratory support apparatus, the controller 13 does not control the gas flow through the apparatus. The gas flow from the external gas supply may be controlled manually by a user of the apparatus 10, for example by adjusting a flow valve of the external gas supply. The controller of the apparatus 10 may be configured to generate one or more gas flow reminders and/or alarms, when the high temperature mode is initiated, or shortly after initiation. The gas flow reminders are configured to remind the user to ensure that the flow rate is within the allowable range for operation in high-temperature mode. This may or may not require a reduction in the gas flow from the external gas supply. One or more alarms may be activated, for example after a predetermined time after initiation of the high-temperature mode, should the gas flow not be reduced by the user. One or more flow sensors may be provided in order to measure the gas flow rate from the or each external gas(es) supply, the flow sensors being used by the controller to generate the one or more reminders and/or alarms. When high-temperature mode is terminated, for the reasons described above, it remains important that the flow rate of gases delivered to the patient remains with a safe flow range, to ensure that the residual energy in the system does not cause a temperature spike in the gases flow. Consequently, during cool-down mode, the controller of the apparatus 10 may be configured to generate one or more gas flow reminders and/or alarms, when the high temperature mode is terminated. The gas flow reminders are configured to remind the user ensure that the flow rate should be controlled to remain within the allowable range for operation in cool-down mode. This may require the gas flow from the external gas supply to be maintained for a predetermined time period, or at least maintained above a minimum threshold. One or more alarms may be activated, for example after a predetermined time after initiation of the high-temperature mode, should the gas flow drop below an allowable range or minimum threshold.

We refer above to control of the electrical energy supplied to the humidifier heater. The humidifier heater is the primary mechanism by which the gases flow is heated. It is also possible that the patient conduit that delivers the gases flow from the humidifier to the patient interface is also heated, via a conduit heater. The algorithms described above may also control the conduit heater as part of a high-temperature mode and a cool-down mode.

There are many techniques for controlling the electrical energy supplied to the humidifier heater. One of these is pulse wave modulation (PWM), as referred to in the examples above. PWM can be controlled during a high-temperature mode, and a cool-down mode, to provide control of the heating and humidification of the gases flow as described above. A duty cycle of the humidifier heater may be similarly controlled. This disclosure envisages any other technique could be used to control the electrical power to the humidifier heater. The humidifier heater will typically comprise a heater plate, although other types of humidifier heater are envisaged.

With reference to Figure 7, Apparatus 10 can be configured to be in communication with a remote management system 101, that may hosted in one or more servers. The remote management system 101 may be cloud based, or hosted in a network of the hospital or the like in which the apparatus 10 is used.

Remote management system 101 may by in communication with a remote device 103, such as a smart phone, tablet, laptop or any other type of remote electronic processing device.

The apparatus 10, remote management system 101 and remote device 103 can be in communication via wired or wireless communication interface 105, indicated by the dashed lines in Figure 7.

The remote management system 101 may be used for patient management.

For example, the remote management system 101 may be configured to control one or more aspects of the apparatus 10 remotely, and/or may be configured to receive and/or transmit data between the apparatus 10 and the remote management system 101 and the remote device 103.

The remote management system may be configured to control controller 13 to control one or more aspects of the operation of the apparatus. Such aspects include but are not limited to: operating the flow generator 11 to create a flow of gas (gases flow) for delivery to a patient, operating the humidifier 12 (if present) to humidify and/or heat the generated gases flow, controlling a flow of oxygen into the flow generator blower, receiving user input from the remote device 103 for reconfiguration and/or user-defined operation of the apparatus 10, and outputting information to the remote device 103.

**In** one embodiment, the remote management system 101 only receives usage data and presents and/or stores that information. For example, the remote management system 101 may store data indicative of use of the apparatus in high-temperature mode.

Alternatively, the remote management system 101 receives, displays and/or stores patient data and usage data and therapy data. This information is accessible by a physician or patient or other party.

Optionally, the remote management system 101 can transmit control data to the apparatus, for example new therapy parameters to the apparatus 100, the apparatus subsequently being operative according to those one or more new therapy parameters.

This sending of control data may be an alternative to receiving data from the apparatus.

The remote management system may receive usage data and/or therapy data directly from the apparatus 10 via the communication interface 105.

The usage data may include:
a. the amount of time the apparatus 10 is used in a given time period;
b. the length of each period of use;
c. patient compliance with a specified therapy. For example, such data could include the number of minutes/hours/days that the patient is receiving therapy; therapy start time; therapy end time.

The therapy data may include:
a. one or more therapy parameters, for example humidity or dew-point or flow rate, or SpO2, or parameters related to the high temperature mode.
b. the number of times the high temperature mode was used for and/or
c. the number of times the high temperature mode is used per day.

Usage data and therapy data may be transmitted to the remote management system 101 at the end of each therapy session, or at least once every 24 hours. For example, usage data and therapy data may be transmitted a predetermined time after therapy has finished. In one example usage data and therapy data may be transmitted during a drying mode that is activated after therapy is finished. The usage data and therapy data may be transmitted to the remote device 101, which could be the patient's phone for example.

Optionally after the high-temperature mode has terminated, the apparatus 10 may be controlled according to a drying mode. For example, if the patient presses an OFF button to end therapy, then a drying mode may be activated.

In drying mode, the apparatus 10 generates a message instructing the patient and/or user not to use the apparatus 10. For example a message may comprise an instruction on the screen of the user interface 14, or instructions on remote device 103.

The drying mode can be operative for a predetermined time, for example between 60 mins and 120 mins, and preferably about 90 mins. In drying mode the humidifier heater plate power is off or reduced to a very low power for example less than 5W, and the conduit heater wire 16c is powered to a maximum. Flow generator 11 is controlled at a set flow rate e.g. between 10 and 30 L/min for the duration of the drying mode. Drying mode dries out condensate that may be formed in the conduit 9. This can be particularly useful as use of the high-temperature mode increases dew-point which could lead to more condensate as the apparatus transitions out of the high- temperature mode.

The communications interface 105 could include a modem. The communications interface 105 could include a Wifi router and/or Bluetooth^{®}.

The remote management system 101 and remote device 103 may be configured to mirror the display of the user interface 14 on the remote device 103 so as to send real-time data to the remote device 103. For example, one, some or all of the various GUIs of the user interface 14 may be mirrored. For example, instructions of reduced flow that are displayed on the user interface 14 may be transmitted and presented on the mobile phone of the patient. This can help with guiding a patient on how to use the high temperature mode. Warnings and/or alarms can also be displayed on the remote device 103. The real-time date may include the countdown timer. The timer may be within the controller 13 of the apparatus 10. Alternatively, the timer could be generated by the remote management system 101 and/or the remote device 103, for example by the patient's phone. For example, a clock or timer function of the mobile phone can be used to time duration of high temperature mode. This is in case the patient falls asleep or is otherwise distracted whilst the apparatus is operating in high-temperature mode, e.g. watching TV or reading a book. This reminder system ensures the patient remains safe when operating in high-temperature mode.

The patient management system 101 may be configured to receive and store data indicative of the usage of the apparatus 10, for example usage of the high temperature mode. This information may be included in a therapy report that is generated by the patient management system 101, or which is generated remotely from the data stored on the patient management system 101. This report may be sent to a remote device 103 of the patient or may be sent/accessed to a remote device 103 of the patient's clinician/physician.

Alternative cool-down modes are also envisaged as modifications of, or instead of, the cool-down mode described above with reference to Figure 3.

For example, in another example of the cool-down mode, the humidifier heater plate and/or the conduit heater wire 16c are reduced to a low power or zero. The temperature at the patient end of the conduit 9 is measured using patient end sensor 26. Optionally there may be a temperature sensor 3b at the outlet of the humidifier 12 (for example within the gases path downstream of the humidifier 12 and adjacent the humidifier outlet). Optionally the temperature at the outlet of the humidifier 12 may be measured. Once either the measured patient end temperature is at 37 °C or below, the flow generator 11 may be controlled such that the flow rate may be changed to a set flow rate set by the user. Alternatively, once the temperature at the outlet of the humidifier 12 is at or below 37 °C then the flow rate is changed.

This modified cool-down mode is a modification of step 302 described above with reference to Figure 3. In this modified cool-down mode, the flow rate is not changed until the temperature (at the patient end of the conduit and/or at the outlet of the humidifier 12) falls below a predetermined threshold.

In another possible modification to cool-down mode step 302, the flow rate may not be ramped once high-temperature mode is exited. This could be because the set flow rate i.e. the therapeutic flow rate, has not been changed. In that case the humidifier heater plate and/or conduit heater 16c power is either set to 0 or reduced to a low power , for example less than 5W, to allow the temperature to reduce.

It is envisaged that the selectable flow rate may only be expanded to the normal mode flow range once the temperature at the patient end and/or at the outlet of the humidifier 12 is less than a safe temperature threshold, for example 37 °C.

It is also envisaged that during cool-down mode, once high-temperature mode is finished, the apparatus 10 will default to providing a humidity setting of 37 °C dew point to the patient. Humidity settings may be locked when in high-temperature mode so that the user cannot change these humidity settings. Once the timer expires or once a user ends high-temperature mode, the humidity settings may be changeable. If the user changes the humidity setting to a lower humidity setting than 37 °C dew point e.g. 34 °C dew point or 31 °C dew point, then the apparatus 10 is configured to reduce the humidifier heater plate power and/or the heater wire 16c power (or switch off power) until at least the patient end temperature, as measured by patient end sensor 26, reads a temperature that corresponds to the current set humidity setting. Alternatively, the controller 13 measures the humidifier chamber outlet temperature using sensor 3b, until that has reached the set humidity. Once the temperature is at the current setting, normal humidity control (i.e. normal control of humidifier heater plate power and/or the heater wire 16c) is resumed.

An apparatus and methodology in accordance with this disclosure advantageously enables respiratory gases to be safely delivered to a patient at higher temperatures than prior art respiratory therapy apparatus, to treat respiratory illness, particularly illness of the upper respiratory tract, caused by viruses and/or bacteria. The high-temperature mode in accordance with this disclosure delivers gases with higher energy than in normal mode i.e. normal therapy mode. The higher energy of gases provides higher energy to the upper airways of the patient. The higher energy can cause denaturing of the viruses and/or restrict or stop virus replication. This in turn can reduce symptoms and can improve patient condition. An apparatus and methodology in accordance with this disclosure also facilitates a safe transition of the gases flow from high-temperature mode back to normal mode. Further, the flow rate range when in high-temperature mode may be limited, which provides a safe high-temperature mode, and reduces the likelihood of over enthalpy or over energising the patient. Further, safety features are provided to keep the patient safe, even after a high-temperature mode is terminated.

An apparatus and methodology in accordance with this disclosure also provides one or more safety features to enable such high temperatures gases to be delivered safely, and further, to control the increased energy residual in the system once high-temperature mode is terminated.

International Application No. PCT/NZ2017/050119, titled "Thermistor Flow Sensor Having Multiple Temperature Points", filed on September 13, 2017, International Application No. PCT/NZ2016/050193, titled "Flow Path Sensing for Respiratory support Apparatus", filed on December 2, 2016, and US patent US8453641 filed 19 August 2005, are hereby incorporated by reference in their entireties.

Reference to any prior art in this specification is not, and should not be taken as, an acknowledgement or any form of suggestion that the prior art forms part of the common general knowledge in the field of endeavour in any country in the world.

Where reference is used herein to directional terms such as 'up', 'down', 'forward', 'rearward', 'horizontal', 'vertical' etc., those terms refer to when the apparatus is in a typical in-use position, and are used to show and/or describe relative directions or orientations.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

The terms "approximately," "about," and "substantially" as used herein represent an amount close to the stated amount that still performs a desired function or achieves a desired result. For example, in some embodiments, as the context may permit, the terms "approximately", "about", and "substantially" may refer to an amount that is within less than or equal to 10% of, within less than or equal to 5% of, and within less than or equal to 1% of the stated amount.

Reference to any prior art in this specification is not, and should not be taken as, an acknowledgement or any form of suggestion that that prior art forms part of the common general knowledge in the field of endeavour in any country in the world.

The disclosed apparatus and systems may also be said broadly to consist in the parts, elements and features referred to or indicated in the specification of the application, individually or collectively, in any or all combinations of two or more of said parts, elements or features.

Where, in the foregoing description reference has been made to integers or components having known equivalents thereof, those integers are herein incorporated as if individually set forth.

Depending on the embodiment, certain acts, events, or functions of any of the algorithms, methods, or processes described herein can be performed in a different sequence, can be added, merged, or left out altogether (for example, not all described acts or events are necessary for the practice of the algorithms). Moreover, in certain embodiments, acts or events can be performed concurrently, for example, through multi-threaded processing, interrupt processing, or multiple processors or processor cores or on other parallel architectures, rather than sequentially.

It should be noted that various changes and modifications to the presently preferred embodiments described herein will be apparent to those skilled in the art. Such changes and modifications may be made without departing from the spirit and scope of the disclosed apparatus and systems and without diminishing its attendant advantages. For instance, various components may be repositioned as desired. It is therefore intended that such changes and modifications be included within the scope of the disclosed apparatus and systems. Moreover, not all of the features, aspects and advantages are necessarily required to practice the disclosed apparatus and systems. Accordingly, the scope of the disclosed apparatus and systems is intended to be defined only by the claims that follow.
The present disclosure extends to the following numbered clauses:
1. A respiratory support apparatus configured to provide a gases flow to a patient, the respiratory support apparatus comprising:
   a flow generator configured to generate the gases flow;
   a humidifier configured to humidify the gases flow;
   a controller, the apparatus being controlled by the controller to function in at least two modes, being a normal mode and a high-temperature mode, wherein when in the high-temperature mode, the peak temperature of gases delivered to the patient is higher than the peak temperature of gases delivered to the patient when in the normal mode;
   wherein in the normal mode the controller controls the flow generator to generate the gases flow at a flow rate within a first flow rate range that is limited by a first peak flow rate; and
   wherein in the high-temperature mode, the controller controls the flow generator to generate the gases flow at a flow rate within a second flow rate range that is limited by a second peak flow rate, the second peak flow rate being lower than the first peak flow rate of the normal mode.
2. The apparatus of clause 1 wherein the first flow rate range is limited by a first minimum flow rate, and the second flow rate range is limited by a second minimum flow rate, the second minimum flow rate being higher than the first minimum flow rate.
3. The apparatus of any one of the preceding clauses wherein the magnitude of a change of flow rate range between high-temperature mode and normal mode is controlled so as to be below a predetermined threshold.
4. The apparatus of clause 2 wherein the minimum flow rate of the second flow rate range, is equal to or less than the minimum flow rate of the first flow rate range.
5. The apparatus of any one of the preceding clauses wherein the second flow rate range is narrower than the first flow rate range.
6. The apparatus of any one of the preceding clauses wherein the second flow rate range is narrower than the first flow rate range by between 1 and 70 L/min, preferably between 5 and 60 L/min and more preferably between 10 and 60 L/min.
7. The apparatus of any one of the preceding clauses wherein when in the high-temperature mode the gases flow to the patient is controlled to a temperature range of 41 to 50 °C, preferably 43 to 47 °C.
8. The apparatus of any one of the preceding clauses wherein the controller limits the duration of the high-temperature mode to a set duration.
9. The apparatus of any one of the preceding clauses and configured to be controlled by the controller in a further mode, being a cool-down mode, the cool-down mode being operative after the high-temperature mode, such that when in the cool-down mode, any one or more of:
   a. the flow rate is controlled to a flow-rate set point, wherein the rate of change of the flow rate from the flow rate used during high-temperature mode to the flow rate set point is controlled to be below a predetermined threshold;
   b. the electrical power supplied to the humidifier heater is reduced from that used during normal mode and/or high-temperature mode; and/or
   c. a temperature set point of the gases flow delivered to the patient is reduced from that during high-temperature mode.
10. A respiratory support apparatus configured to provide a gases flow to a patient, the respiratory support apparatus comprising:
   a flow generator configured to generate the gases flow;
   a humidifier configured to humidify the gases flow;
   a controller, the apparatus being controlled by the controller to function in at least two modes, being a normal mode and a high-temperature mode, wherein when in the high-temperature mode, the temperature of gases delivered to the patient is higher than the temperature of gases delivered to the patient in normal mode;
   wherein in the high-temperature mode the controller limits the duration of the high-temperature mode to a maximum duration.
11. The apparatus of clause 10 wherein the controller limits the duration of the high-temperature mode using a signal generated by a timer.
12. The apparatus of clause 11 wherein the set duration is between 15 and 180 minutes, preferably 20 and 120 minutes, and more preferably between 30 and 90 minutes.
13. The apparatus of clause 10 wherein the controller limits the duration of the high-temperature mode using a signal generated by a counter.
14. The apparatus of any one of clauses 11 to 13 wherein the apparatus generates the signal.
15. The apparatus of clause 14 wherein the apparatus comprises a further controller, configured to generate the signal.
16. The apparatus of any one of clauses 11 to 13 wherein the apparatus receives the signal from a remote device.
17. The apparatus of any one of clauses 12 to 15 wherein the apparatus is configured to be controlled by the controller in a further mode, being a cool-down mode, the cool-down mode being operative after the high-temperature mode ends.
18. The apparatus of clause 17 wherein, when in the cool-down mode, any one or more of:
   a. the flow rate is controlled to a flow rate set point, wherein the rate of change of the flow rate from the flow rate used during high-temperature mode to the flow rate set point is controlled to be below a predetermined threshold;
   b. the electrical power supplied to the humidifier heater is reduced from that used during normal mode and/or high-temperature mode; and/or
   c. a temperature set point of the gases flow delivered to the patient is reduced from that during high-temperature mode.
19. The apparatus of any one of the preceding clauses wherein in the cool-down mode, the apparatus is controlled to a gases flow temperature set point of 40 °C or less.
20. The apparatus of any one of the preceding clauses wherein in the cool-down mode, the apparatus is controlled to generate a gases flow at a flow rate within the first range of flow rates that are generated in the normal mode.
21. The apparatus of any one of the preceding clauses wherein in the cool-down mode, the apparatus is controlled such that the enthalpy and/or dew-point of the gases flow is reduced from that of the high-temperature mode.
22. The apparatus of any one of the preceding clauses wherein when in the high-temperature mode, the gases flow to the patient is in a temperature range of 41 to 50 °C, preferably 43 to 47 °C.
23. A respiratory support apparatus configured to provide a gases flow to a patient, the respiratory support apparatus comprising:
   a flow generator configured to generate the gases flow;
   a humidifier configured to humidify the gases flow, the humidifier comprising a humidifier heater;
   a controller, the apparatus being controlled by the controller to function in at least two modes, being a normal mode and a high-temperature mode, wherein when in the high-temperature mode, the temperature of gases delivered to the patient is higher than the temperature of gases delivered to the patient in the normal mode;
   the apparatus further being controlled by the controller in a further mode, being a cool-down mode, the cool-down mode being operative after the high-temperature mode ends, such that when in the cool-down mode, any one or more of:
      a. the flow rate is controlled to a flow rate set point, wherein the rate of change of the flow rate from the flow rate used during high- temperature mode to the flow rate set point is controlled to be below a predetermined threshold;
      b. the electrical power supplied to the humidifier heater is reduced from that used during normal and/or high-temperature mode; and/or
      c. a temperature set point of the gases flow delivered to the patient is reduced from that during high-temperature mode;
      d. the flow rate, and/or the rate of change of the flow rate from the flow rate used during high-temperature mode, is controlled according to a predetermined flow-time curve/relationship.
24. The apparatus of clause 23 wherein the cool-down mode is automatically selected by the controller, in response to one or more predetermined conditions of operation of the apparatus.
25. The apparatus of clause 24 wherein the one or more predetermined conditions of operation are selected from any one or more of:
   a. a predetermined period of time, as determined by a timer;
   b. a predetermined number of counts, as determined by a counter;
   c. a gases flow dew-point being exceeded;
   d. a gases flow enthalpy being exceeded;
   e. the user exiting high-temperature mode.
26. The apparatus of any one of clauses 23 to 25 wherein the controller returns the apparatus to normal mode, after the cool-down mode.
27. The apparatus of clause 26 wherein the controller returns the apparatus to normal mode after a predetermined period of time, which may be in the range of 15 to 60 minutes, or 30 minutes.
28. The apparatus of clause 26 wherein the controller returns the apparatus to normal mode in dependence upon the temperature of the gases flow.
29. The apparatus of clause 28 wherein the controller returns the apparatus to normal mode in dependence upon the temperature of the gases flow being less than a predetermined temperature.
30. The apparatus of clause 26 wherein the controller returns the apparatus to normal mode in dependence upon the dew-point of the gases flow.
31. The apparatus of clause 26 wherein the controller returns the apparatus to normal mode in dependence upon the enthalpy of the gases flow.
32. The apparatus of any one of clauses 22 to 31 wherein when in the high-temperature mode, the gases flow to the patient is in a temperature range of 41 to 50 °C, preferably 43 to 47 °C.
33. A respiratory support apparatus configured to provide a gases flow to a patient, the respiratory support apparatus comprising:
   a flow generator configured to generate the gases flow;
   a humidifier configured to humidify the gases flow;
   a controller, the apparatus being controlled by the controller to function in at least two modes, being a normal mode and a high-temperature mode, wherein when in the high-temperature mode, the temperature of gases delivered to the patient is higher than the temperature of gases delivered to the patient in normal mode;
   wherein the controller controls the apparatus to restrict a characteristic of the gases flow during high-temperature mode, and/or immediately after termination of high-temperature mode, such that the enthalpy and/or the dew-point of the gases flow is below a predetermined level.
34. The apparatus of any one of the preceding clauses wherein when in the high-temperature mode, the gases flow to the patient is in a temperature range of 41 to 50 °C, preferably 43 to 47 °C.
35. A respiratory support apparatus configured to provide a gases flow to a patient, the respiratory support apparatus comprising:
   a flow generator configured to generate the gases flow;
   a humidifier configured to humidify the gases flow, the humidifier comprising a humidifier heater;
   a controller, the apparatus being controlled by the controller to function in at least two modes, being a normal mode and a high-temperature mode, wherein when in the high-temperature mode the dew-point of gases delivered to the patient is higher than the dew-point of gases delivered to the patient in normal mode; wherein when in the high-temperature mode, the gases flow to the patient is in a dew-point range of 43 to 50 °C.
36. An apparatus according to any one of the preceding clauses comprising any one or more of:
   a. a patient interface configured to be mounted on the patient and to receive the humidified gases flow from the humidifier;
   b. a patient circuit comprising at least one patient conduit configured to be connected to an outlet of the humidifier, and to receive the humidified gases flow from the humidifier;
   c. headgear configured to mount a patient interface on the patient's head.
37. The apparatus of clause 36 wherein the patient interface comprises a nasal cannula.
38. The apparatus of any one of the preceding clauses, comprising a user interface arranged to a receive an input from the user to control the apparatus.
39. The apparatus of clause 38 wherein the user interface is a graphic user interface, and preferably a touch screen graphic user interface.
40. The apparatus of clause 38 or 39 wherein the normal and high-temperature modes are user selectable, via the user interface.
41. The apparatus of any one of the preceding clauses wherein the apparatus is controlled to provide a cool-down mode, after termination of the high-temperature mode.
42. The apparatus of clause 41 wherein the cool-down mode is automatically selected by the controller, in response to one or more predetermined conditions of operation of the apparatus.
43. The apparatus of clause 42 wherein the one or more predetermined conditions of operation are selected from any one or more of:
   a. a predetermined period of time, as determined by a timer;
   b. a predetermined number of counts, as determined by a counter;
   c. a gases flow dew-point being exceeded;
   d. a gases flow enthalpy being exceeded;
   e. the user exiting high-temperature mode.
44. The apparatus of any one of clauses 41 to 43, wherein the apparatus is controlled, after the cool-down mode, to return to the normal mode.
45. The apparatus of any one of the preceding clauses wherein when in normal mode, the gases flow supplied to the patient is controlled to have any one or more of the following characteristics:
   a. a dew-point in the range of 31 °C - 37 °C;
   b. a temperature in the range of 31 °C - 37 °C;
   c. a flow rate in the range of 10 L/min - 70 L/min.
46. The apparatus of any one of the preceding clauses wherein when in high-temperature mode, the gases flow supplied to the patient is controlled to have any one or more of the following characteristics:
   a. a dew-point in the range of 40 °C - 45 °C;
   b. a temperature in the range of 41 °C - 50 °C;
   c. a flow rate in the range of 30 L/min - 40 L/min;
   d. a minimum flow rate that is a predetermined amount less than the minimum flow rate in the normal mode;
   e. a minimum flow rate that is a predetermined amount more than the minimum flow rate in the normal mode; and
   f. a maximum flow rate that is a predetermined amount less than the maximum flow rate in the normal mode.

## Claims

1. A respiratory support apparatus configured to provide a gases flow to a patient, the respiratory support apparatus comprising:
a flow generator configured to generate the gases flow;
a humidifier configured to humidify the gases flow;
a controller, the apparatus being controlled by the controller to function in at least two modes, being a normal mode and a high-temperature mode, wherein when in the high-temperature mode, the temperature of gases delivered to the patient is higher than the temperature of gases delivered to the patient in normal mode;
wherein in the high-temperature mode the controller limits the duration of the high-temperature mode to a maximum duration.

2. The apparatus of claim 1 wherein the controller limits the duration of the high-temperature mode using a signal generated by a timer.

3. The apparatus of claim 2 wherein the set duration is between 15 and 180 minutes, preferably 20 and 120 minutes, and more preferably between 30 and 90 minutes.

4. The apparatus of claim 1 wherein the controller limits the duration of the high-temperature mode using a signal generated by a counter.

5. The apparatus of any one of claims 2 to 4 wherein the apparatus generates the signal.

6. The apparatus of claim 5 wherein the apparatus comprises a further controller, configured to generate the signal.

7. The apparatus of any one of claims 2 to 4 wherein the apparatus receives the signal from a remote device.

8. The apparatus of any one of claims 3 to 6 wherein the apparatus is configured to be controlled by the controller in a further mode, being a cool-down mode, the cool-down mode being operative after the high-temperature mode ends.

9. The apparatus of claim 8 wherein, when in the cool-down mode, any one or more of:
a. the flow rate is controlled to a flow rate set point, wherein the rate of change of the flow rate from the flow rate used during high-temperature mode to the flow rate set point is controlled to be below a predetermined threshold;
b. the electrical power supplied to the humidifier heater is reduced from that used during normal mode and/or high-temperature mode; and/or
c. a temperature set point of the gases flow delivered to the patient is reduced from that during high-temperature mode.

10. The apparatus of any one of the preceding claims wherein in the cool-down mode, the apparatus is controlled to a gases flow temperature set point of 40 °C or less.

11. The apparatus of any one of the preceding claims wherein in the cool-down mode, the apparatus is controlled to generate a gases flow at a flow rate within the first range of flow rates that are generated in the normal mode.

12. The apparatus of any one of the preceding claims wherein in the cool-down mode, the apparatus is controlled such that the enthalpy and/or dew-point of the gases flow is reduced from that of the high-temperature mode.

13. The apparatus of any one of the preceding claims wherein when in the high-temperature mode, the gases flow to the patient is in a temperature range of 41 to 50 °C, preferably 43 to 47 °C.

14. The respiratory support apparatus of any one of the preceding claims wherein in the normal mode the controller controls the flow generator to generate the gases flow at a flow rate within a first flow rate range that is limited by a first peak flow rate; and
wherein in the high-temperature mode, the controller controls the flow generator to generate the gases flow at a flow rate within a second flow rate range that is limited by a second peak flow rate, the second peak flow rate being lower than the first peak flow rate of the normal mode

15. The respiratory support apparatus of claim 14, wherein at initiation of the high-temperature mode, the apparatus is controlled by the controller to reduce a peak flow rate from the first peak flow rate to the second peak flow rate; and/or wherein at initiation of the high-temperature mode, the apparatus is controlled by the controller to reduce a flow rate range from the first flow rate range to the second flow rate range.
